# EUROPEAN PATENT SPECIFICATION

(11) **EP 2 269 550 B1**
(45) Date of publication and mention of the grant of the patent: **15.05.2013**
(21) Application number: 10167980.1
(22) Date of filing: 30.06.2010
(51) Int. Cl.: A61F 17/00

(54) **Method and device for determining a set of bandages appropriate for a particular injury**
Verfahren und Gerät zur Bestimmung einer Verbandzusammenstellung für eine bestimmte Verletzung
Méthode et outil pour la détermination d'une combinaison des bandages pour une blessure particulière

(30) Priority: 30.06.2009 NL 2003107
(43) Date of publication of application: 05.01.2011
(73) Proprietor: AED Solutions B.V., 6039 RG Stramproy (NL)
(72) Inventor: Henderikx, Hubertus, Henricus, Maria, 6039 RG Stramproy (NL); Borman Raaijen, Francisca, Willemina, Hendrika, 4007 ZK Tiel (NL)
(74) Representative: van Oeffelt, Abraham

(56) References cited:
- WO-A1-01/85566
- WO-A1-03/094811
- WO-A2-2007/061411
- WO-A2-2008/104818
- NL-C2- 1 023 749
- US-A- 5 848 700
- US-A1- 2002 104 774
- US-A1- 2008 237 083
- US-A1- 2009 062 623

## Description

This invention concerns a method and device for determining a set of bandages appropriate for a particular injury, a set of bandages for use with such a method, and a first-aid kit for at least such a set of bandages.

Treating injuries with bandage is a well-known method and has been practised for a long time by professional and non-professional care providers, using dressing material often kept in a first-aid kit. In doing so, the care providers are assumed to be familiar with the dressing material and its specific areas of application and to be able to distinguish the various types of dressing material from one another when they are kept together in a first-aid kit.

In practice, however, it appears that this does not always work out. Care providers are not always properly prepared or trained for their task and, moreover, their concentration may be affected due to the circumstances of the case they are dealing with, leading them to use the wrong dressing material for the injury in question. Furthermore, they may not be able to find particular dressing material in the first-aid kit or that material may not be present in the kit, for instance because it has not been correctly replenished after use.

The international patent application WO 01 85566 describes a first-aid kit that is provided with illustrations with an explanation, which in theory help a user to identify and use the correct dressing material for a particular condition. Even though this solution could in theory lead to a correct choice of type of bandage, in practice it appears that the care provider lacks the peace and quiet to read these instructions and study the drawings, leading to slow or erroneous action.

The United Stated patent application US 2002/104774 describes a first-aid kit that is provided with a guide book, which in theory helps a user to identify and use the correct dressing material for a particular condition. Even though this solution could in theory lead to a correct choice of type of bandage, in practice it appears that the care provider lacks the peace and quiet to read these instructions and study the drawings, leading to slow or erroneous action.

One aim of the invention is therefore to provide a method that ensures that an injury is treated in the correct manner and with the right dressing material.

The invention is defined by the method of claim 1 and the device of claim 5. Preferred embodiments are defined in the dependent claims.

To this end, the invention offers a method for determining a set of bandages appropriate for a particular injury, comprising the steps for providing a decision tree to determine the type of injury on the basis of visual observation of an injured person (symptoms); determine the action to be taken on the basis of the type of injury identified; determine the requisite dressing material on the basis of the action identified; and determine, on the basis of the requisite dressing material thus identified, at least one set of bandages which comprises at least the requisite dressing material. The set of bandages determined in this manner can then be taken from a first-aid kit in order to treat the injury of the person concerned. The steps for treating the person concerned with the aid of the dressing material from the set of bandages may also be included in the decision tree so as to support the care provider after determining which dressing material is needed and taking it from the first-aid kit. The decision tree comprises questions which the care provider can answer with "yes" or "no" on the basis of simple observation, with each answer leading to the next question, which can likewise be answered with "yes" or "no", or to an indication of the set of bandages to be used. For this purpose, the invention provides an audiovisual device for going through the various stages of the decision tree.

The audiovisual device, which guides the care provider through the assistance process and the dressing material options, prevents failure as a result of concentration problems in a practical situation with an injured person, since in such cases care providers appear to be better able to comply with spoken and displayed instructions rather than inferring them by themselves from a text or instructional drawing. As the invention provides an audiovisual device which gives these instructions to the care provider and goes through a decision tree, however, it obviates the need for a remote care provider issuing instructions via an audio or video link.

To enhance the certainty that a care provider will identify the correct type of injury, the decision tree can, on the basis of the established type of injury, generate a control observation to ascertain the accuracy of the type of injury established. Such a control observation includes, for instance, identifying a second symptom of the type of injury established. The control observation can be determined on the basis of a lack of clarity, an inconsistency or a contradiction encountered in going through the decision tree.

Using an audiovisual device in accordance with this invention has the added advantage that the care provider can be guided through this process, the checking stages are gone through unnoticed, and the decision tree can be set up in such a way that any wrong judgement can be rectified by the care provider without arousing suspicion. When the decision tree has been printed out and the care provider notices that he has ended up in an iterative determination, he may be tempted to skip steps, which is prevented by using an audiovisual aid that does not make it obvious to the user in advance what claim he is heading for.

The set of bandages that has been determined and is to be used can be marked in the decision tree for recognition purposes, for instance by means of a letter, icon or colour, ensuring that it can be easily recognised by the care provider. In this case, the same mark will be indicated on the actual set of bandages in the first-aid kit.

The decision tree can be implemented in software that can be run on a device that may or may not have been designed specially for this purpose, e.g. a computer, a PDA or a mobile phone, where existing input facilities may be used, such as a button or microphone, for the answers to be entered by a user to the questions asked. Such a device can guide a care provider through the decision tree by displaying or reading out the questions which the care provider can answer with "yes" or "no" on the basis of simple observation, with each answer leading to the next question, which can likewise be answered with "yes" or "no", or to an indication of the set of bandages to be used.

The questions can in this case be answered via a keyboard or speech recognition for instance. The set of bandages to be used can be described by the device or be indicated by a name or a mark of recognition, e.g. a letter, icon or colour, so that it can be easily recognised by the care provider.

Implementing the decision tree in software makes it possible to tailor the information to the needs of particular users or situations or effectively adapt the decision tree to feedback from users or to dressing material with a changed application or different instructions for use. This makes the device in accordance with the invention usable for a wide range of situations and makes the area of application adaptable, e.g. from a situation where chemicals are used with a serious risk of bums or loss of consciousness to a situation where sports are engaged in and bleeding wounds and fractures may occur. Because of this, the device according to the invention is very flexible and can be used in a wide range of situations.

The device for going through the decision tree may be configured to go through a decision tree focused on an actual emergency situation but may also include a training mode. In the latter case, the instructions will be more extensive and/or there will be more explanation and clarification. Consequently, it is no longer necessary for people undertaking a course to stay in the classroom all day to learn first-aid methods; on the basis of the protocols and the first-aid kit they will be able to learn digitally anywhere and anytime how the various methods have to be carried out. Using a device for going through a decision tree, e.g. Safety Mate, they can thus learn to take protocol-based action. In this case, those doing the course will only have to sit a competency test to qualify. They will then be able to keep their knowledge up to date using Safety Mate.

In a mode intended to be used in a practical situation in which an actual injury has been sustained, the decision tree is optimised to provide instructions as swiftly and simply as possible.

Not only is the device according to the invention thus usable in a wider range of situations, namely in training and practical situations, but the care providers' familiarity with the device can also be enhanced as a result of this, which ensures confidence and calm in a practical situation where the actions to be taken may be identical or at least similar to those learnt in training with the same device.

The area of application or the decision tree stored in the device can be changed simply by replacing a memory or storage medium such as an SD card.

In an additional version, the device for going through the decision tree is integrated in the first-aid kit, which for this purpose is fitted with a display screen such as an LCD, LED or plasma screen. In addition to spoken instructions, the device can thus graphically display the steps for selecting and using the right sets of bandages/injury kits. The screen may be a touch screen on which the user can enter "yes" or "no" in response to the questions displayed simply by touching the appropriate areas on the screen. The screen may also be set to display images of various types or extents of injury to enable the user to identify the relevant injury through optical recognition and image selection, and thus to select the appropriate set of bandages to be used.

The marking used is also applied to the set of bandages so that the care provider can easily find the items thus marked in the first-aid kit. Next, the decision tree can guide the user in applying the dressing material, in which case step-by-step instructions are given instead of or in addition to the questions to be answered with "yes" and "no". In a cheaper version of the device, the dressing material to be used by a care provider in treating at least the most common injuries is subdivided into a number of sets of bandages, e.g. six different ones, and there is a set of accessories with dressing material that is applicable to several types of injury or that is not used up and therefore does not need to be replaced or replenished as frequently. The invention also makes provision for a first-aid kit designed for accommodating such sets of bandages in the form of a number of compartments inside the kit, each of which is designed to hold a specific set, in which case the shape of the sets and the first-aid kit are adapted to each other, including a cheaper version in which the compartments inside the kit are provided with markings or indications to distinguish the various sets of bandages. In yet another version, the first-aid kit has an integrated device to run software in which the decision tree is implemented, giving rise to a first-aid kit that assists a care provider in determining the type of injury and the appropriate bandage for dressing it.

The first-aid kit ideally forms part of a modular care provision system. In a simple version, it comprises only the sets of bandages or injury kits, and in a more elaborate version also a device for going through the decision tree, such as Safety Mate, and in yet another version this device is integrated and the first-aid box is fitted with a display screen. There is an additional version with a defibrillator included or integrated in the first-aid kit, which thus makes up a fully equipped first-aid unit.

Below, the invention is explained in more detail on the basis of a number of figures in which:
- Figure 1 shows a decision tree to determine a set of bandages appropriate for a particular injury;
- Figure 2 shows an overview of dressing material in sets of bandages;
- Figure 3 shows a first-aid kit containing the sets of bandages from figure 2;
- Figure 4 shows an overview of sets of bandages and their applications; and
- Figure 5 shows a device for going through an interactive voice-controlled protocol.

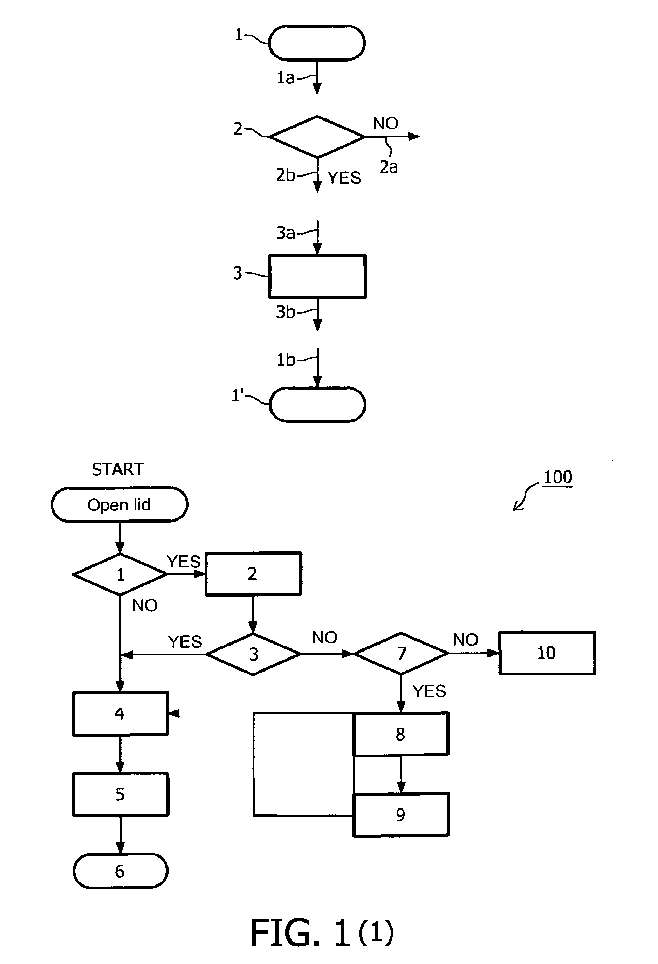

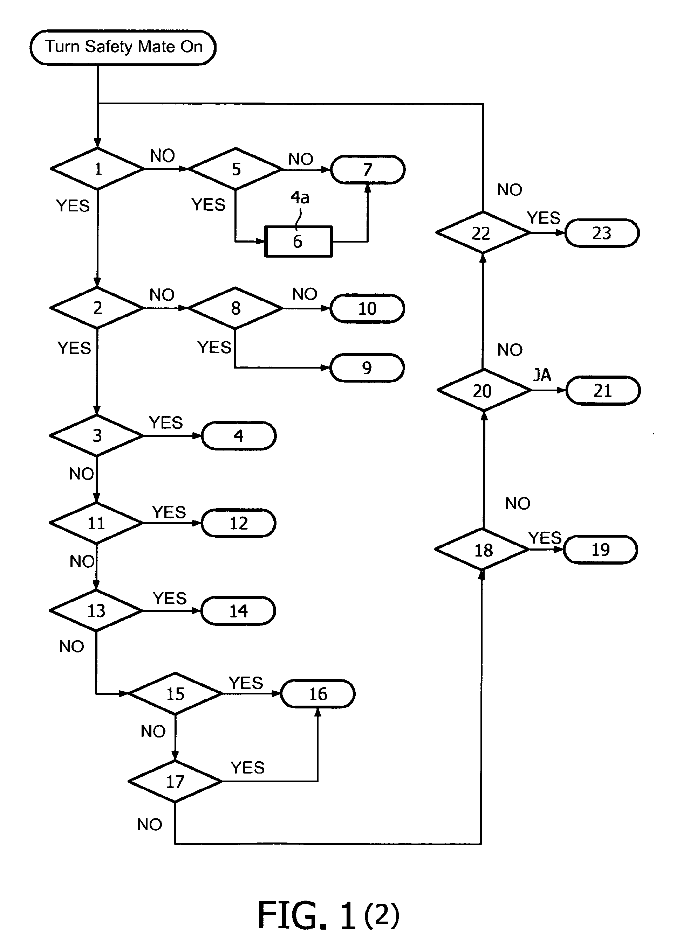

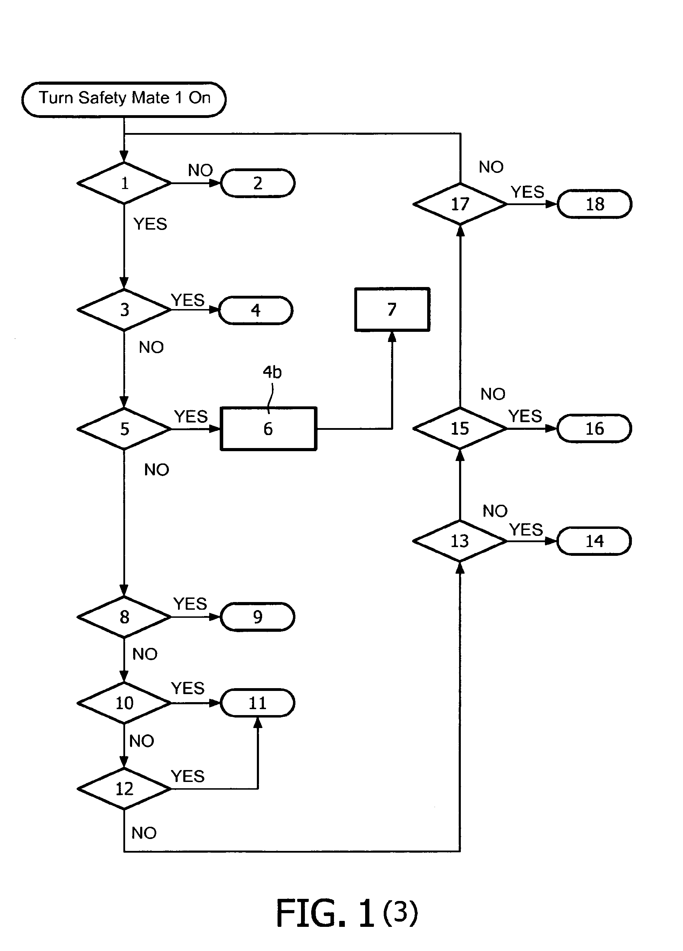

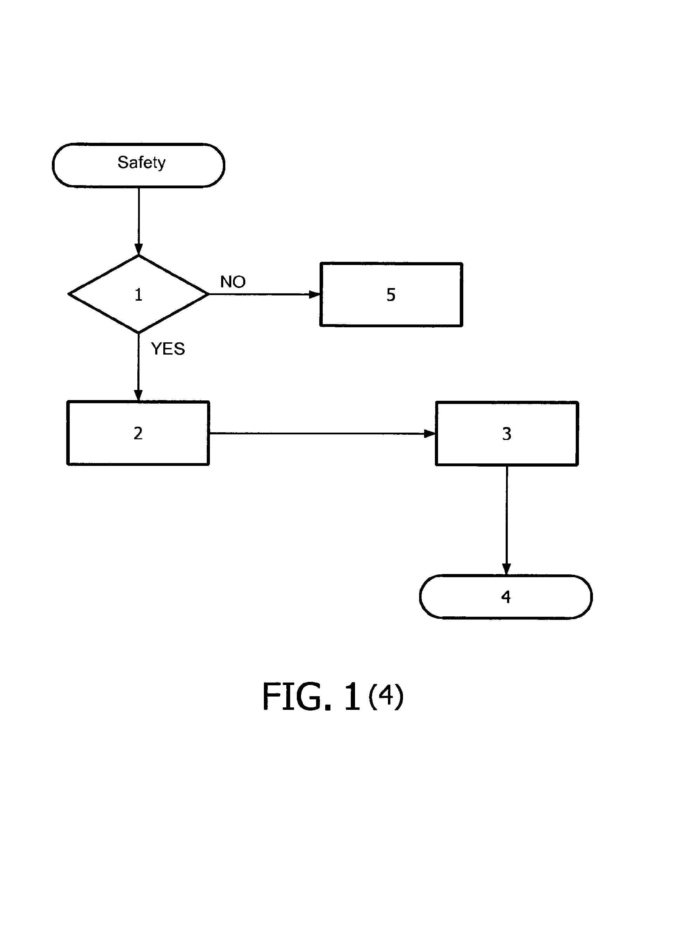

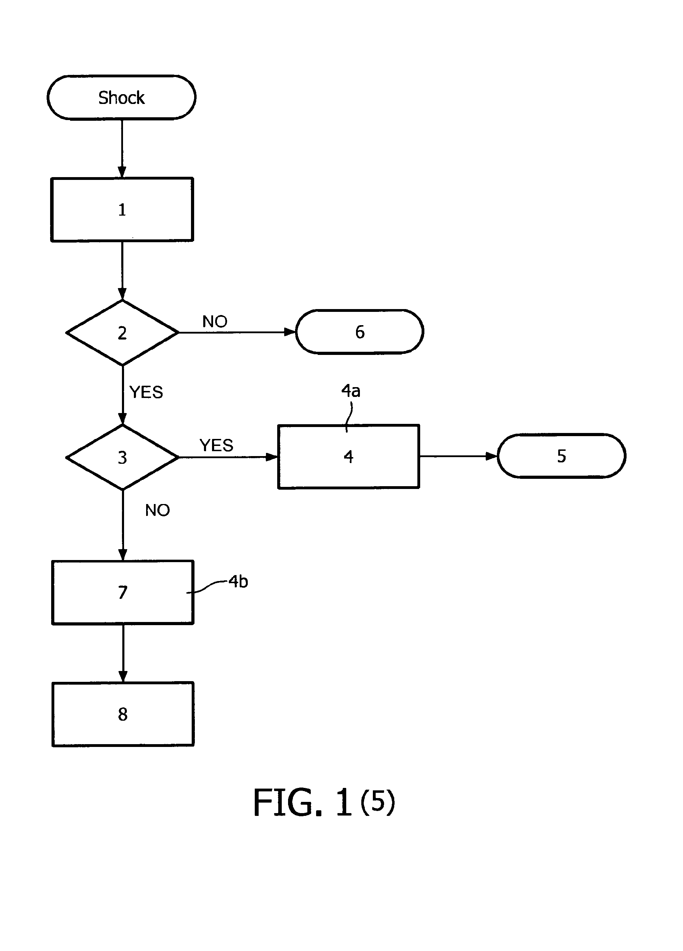

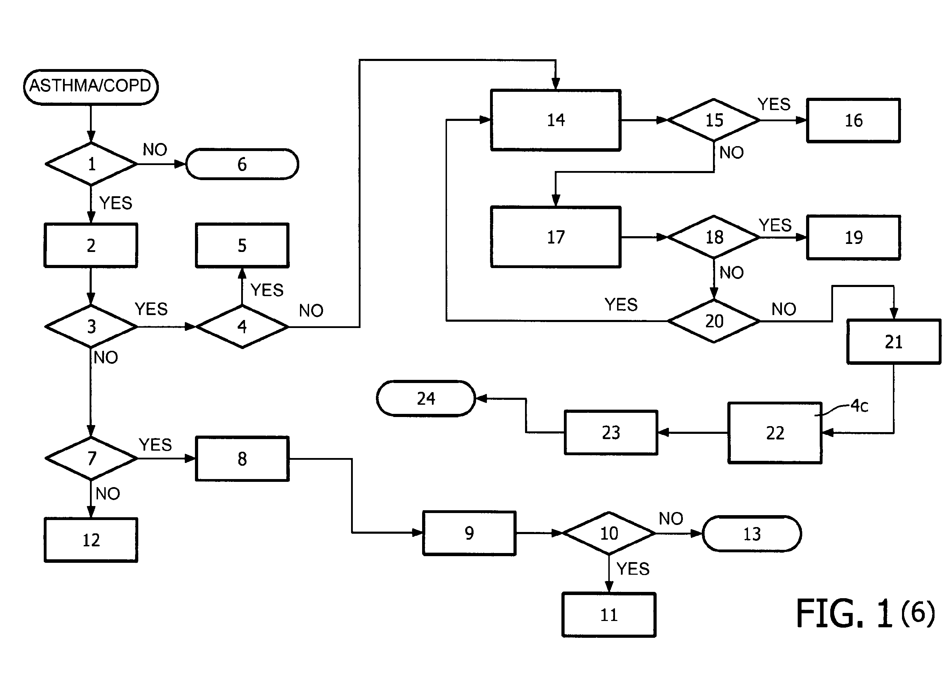

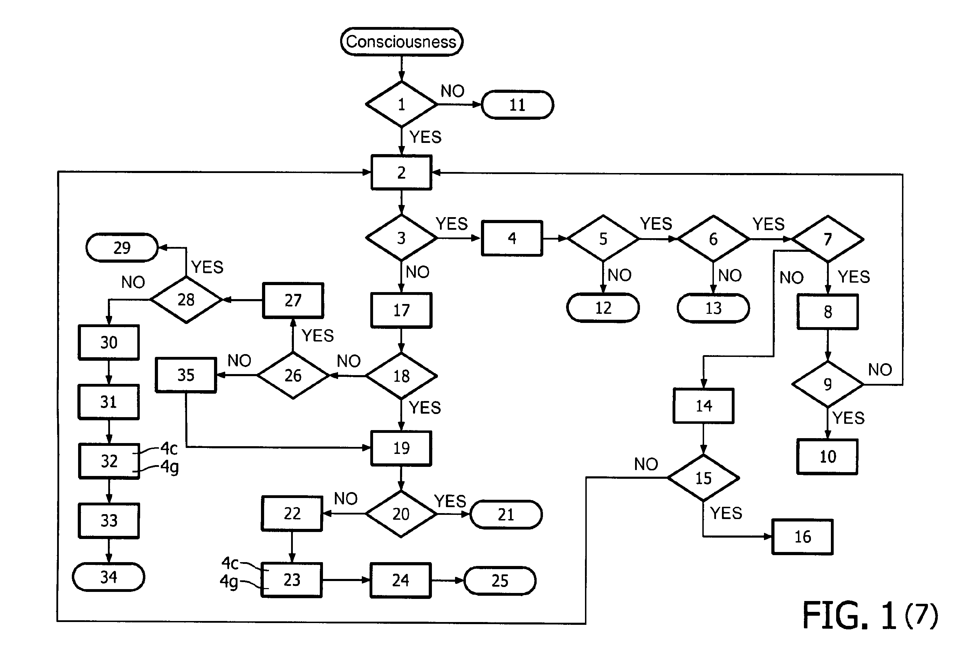

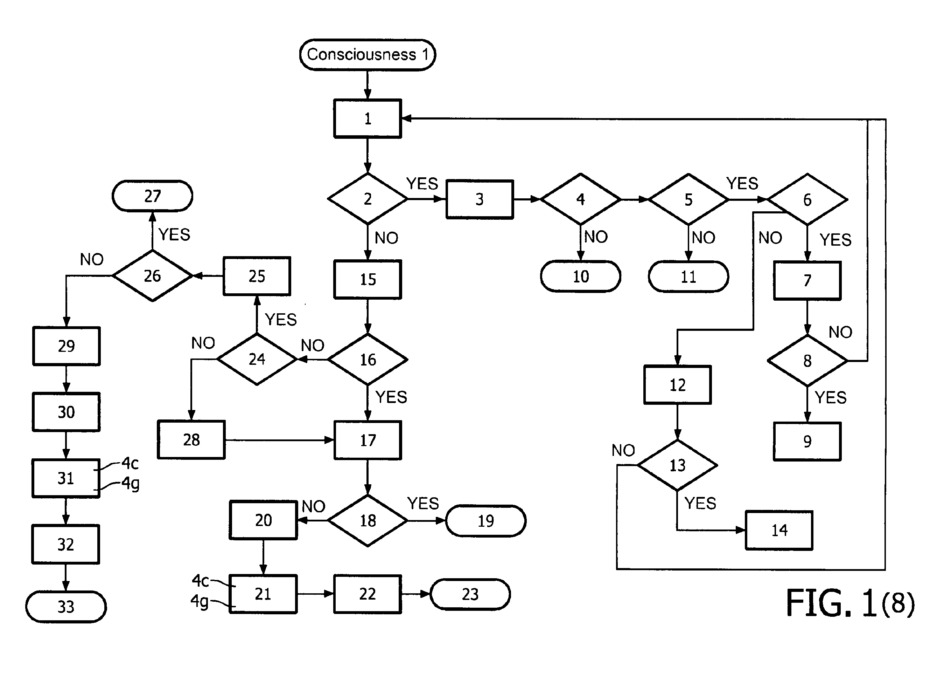

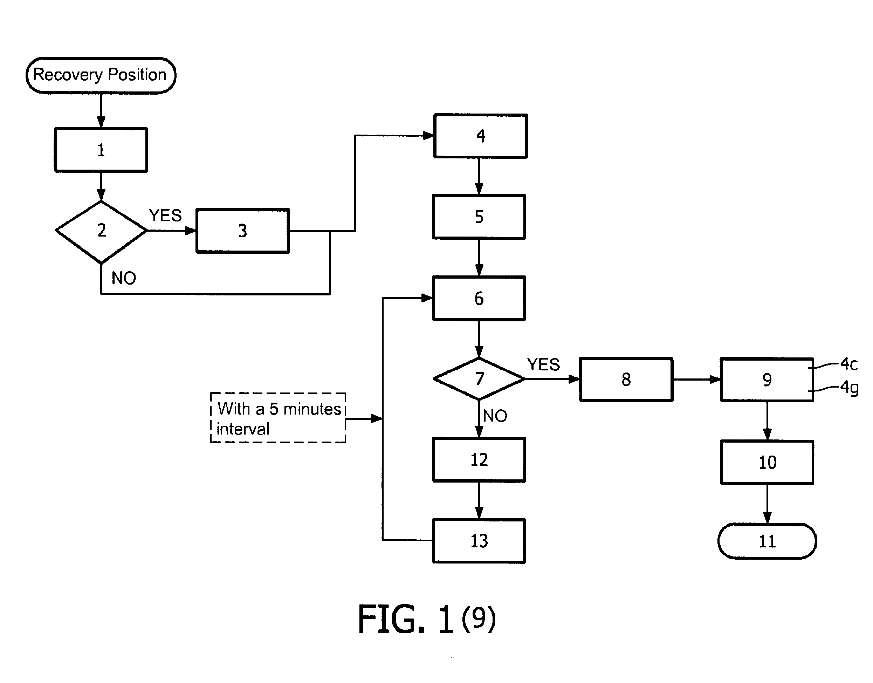

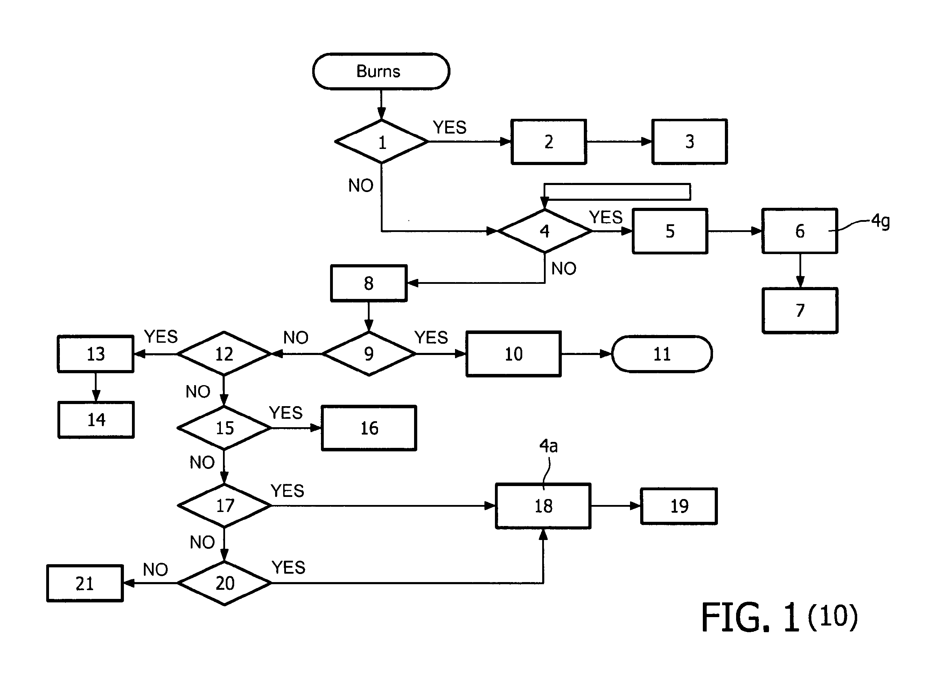

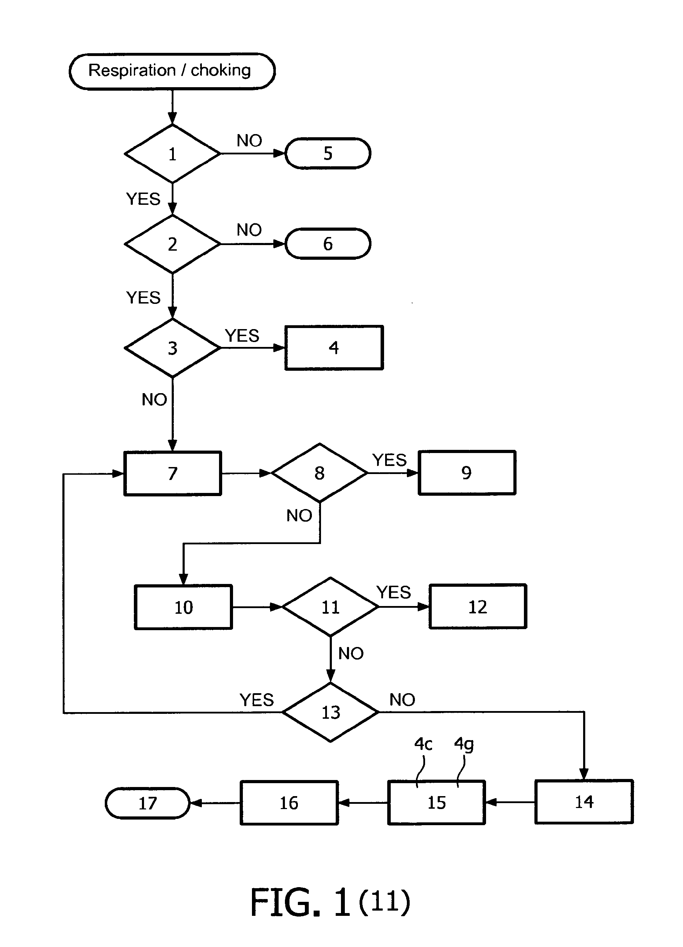

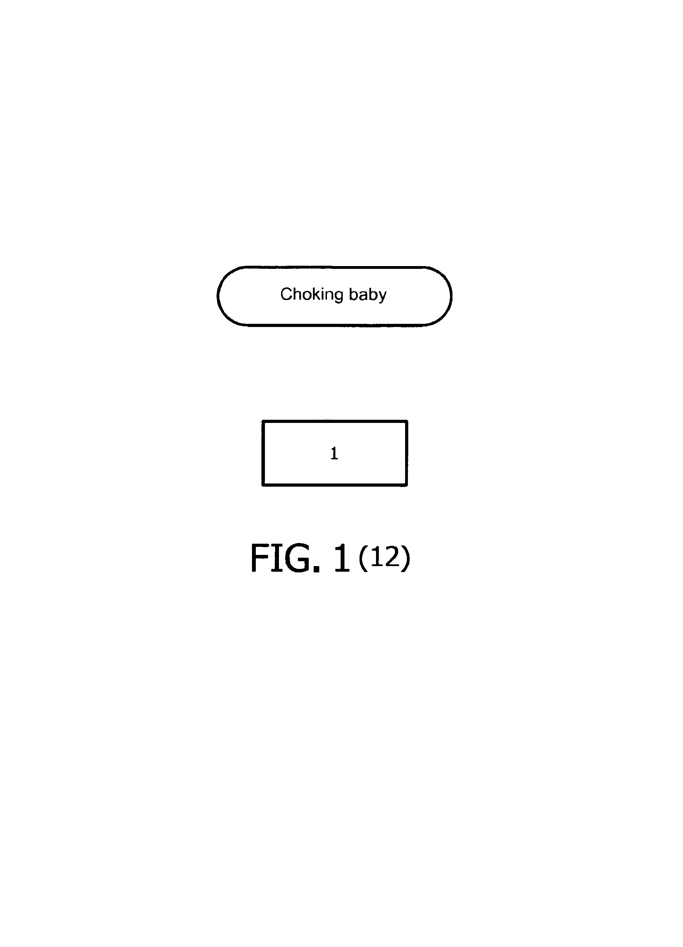

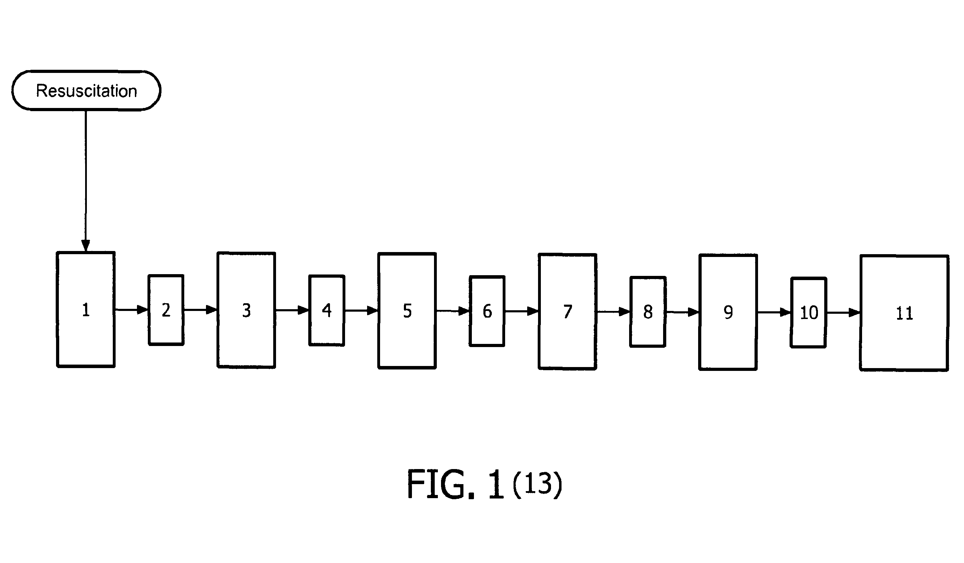

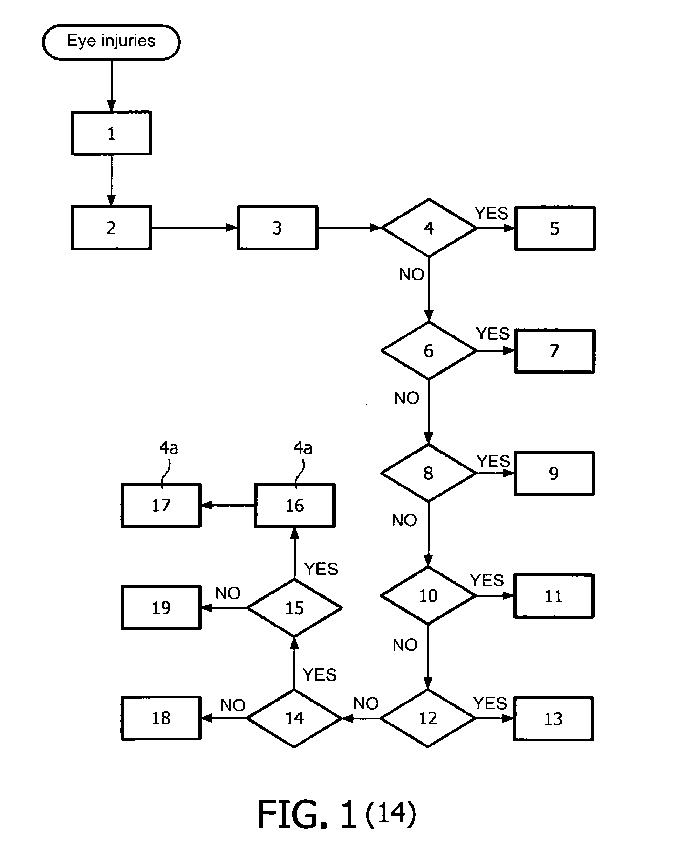

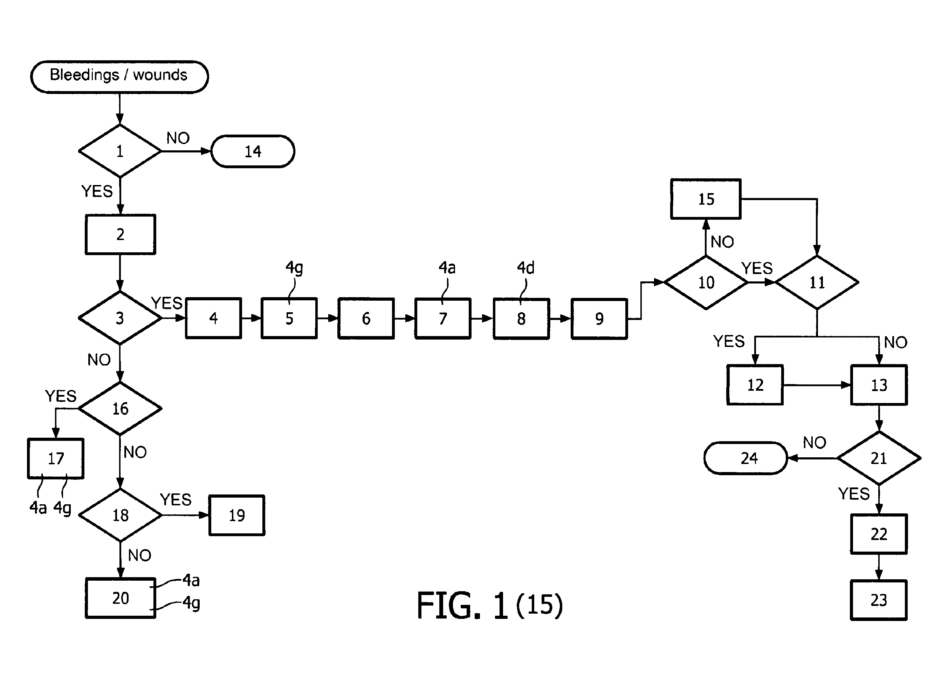

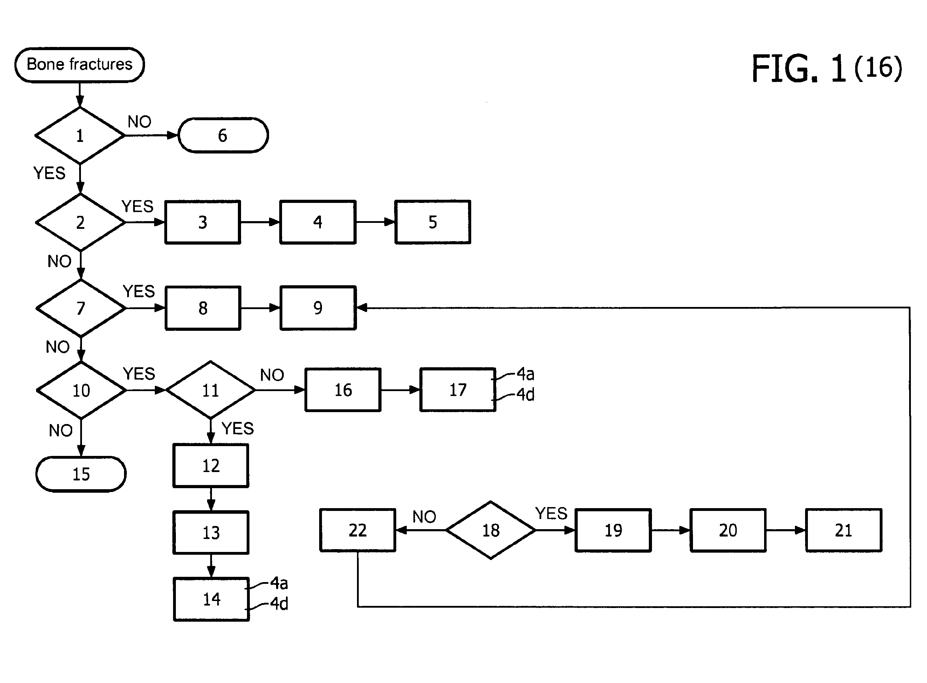

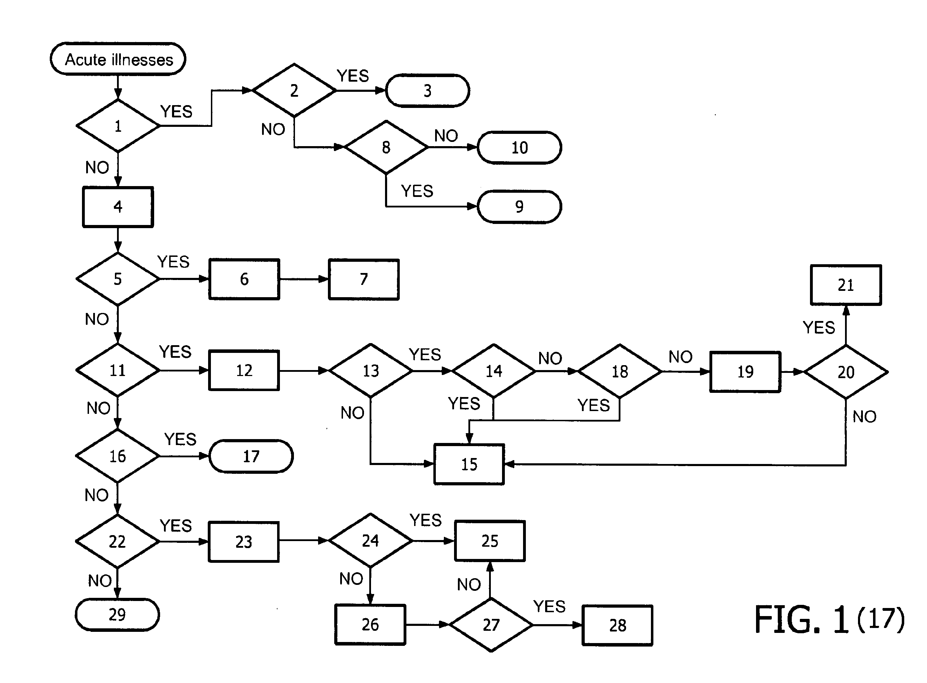

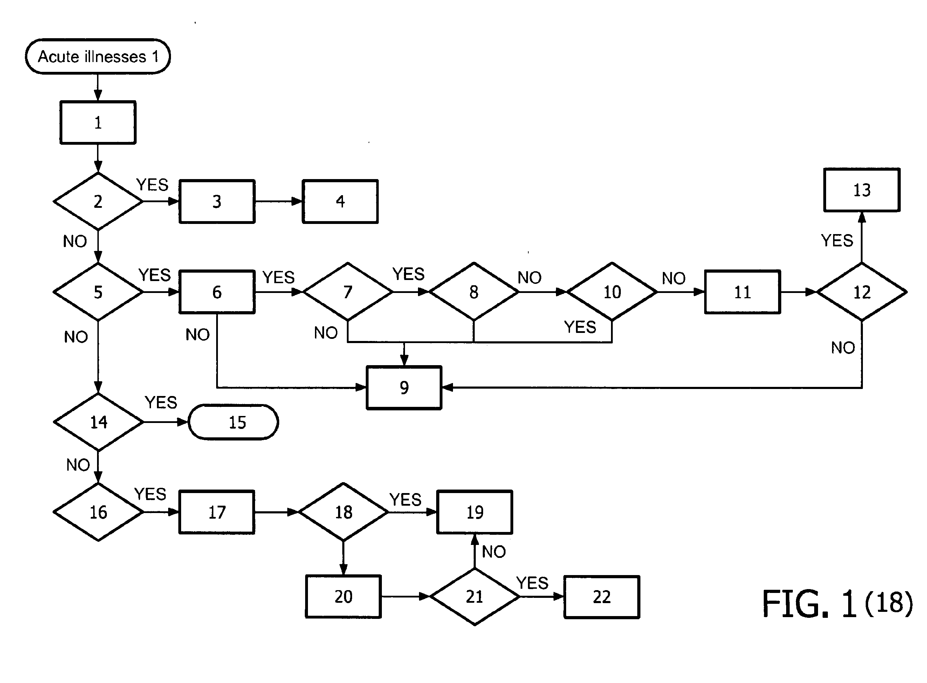

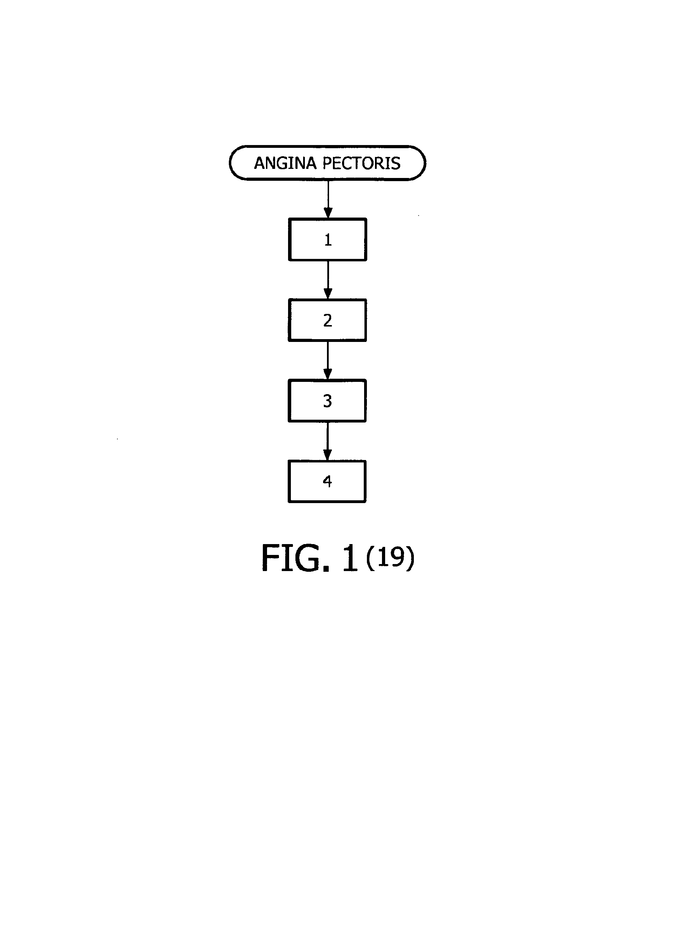

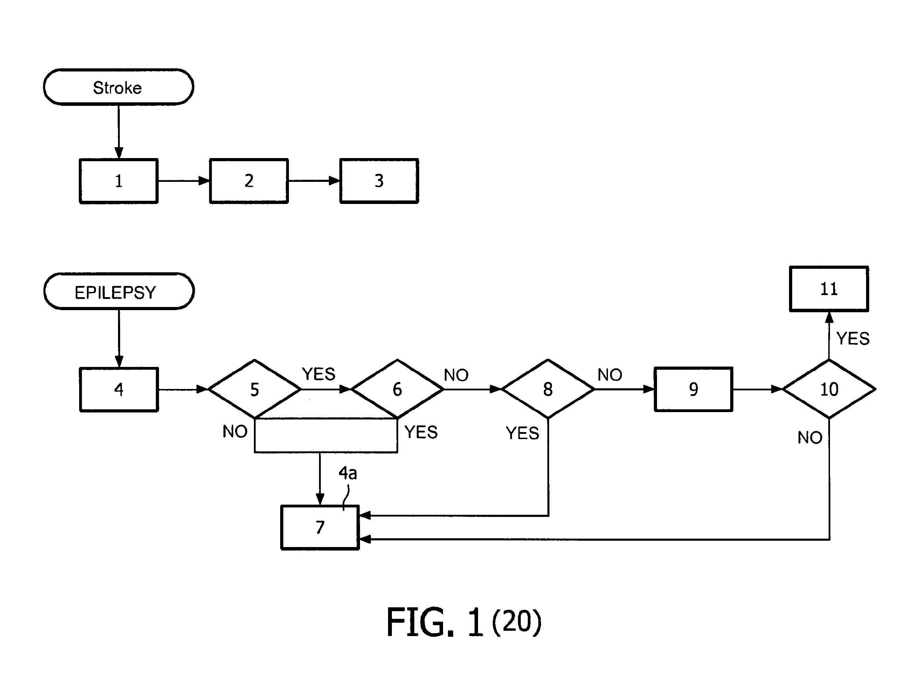

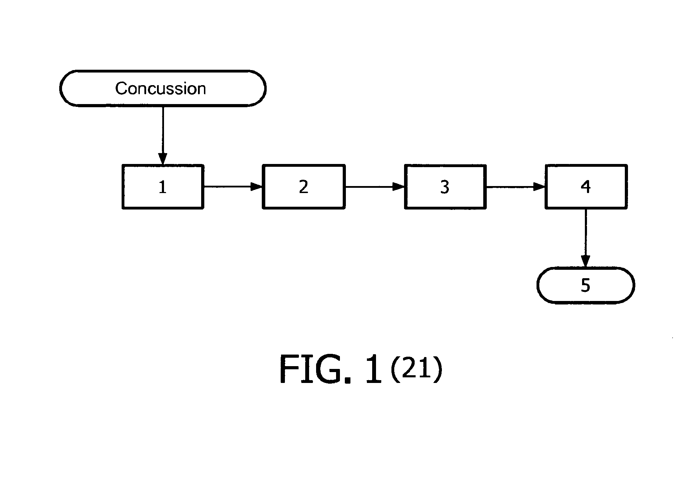

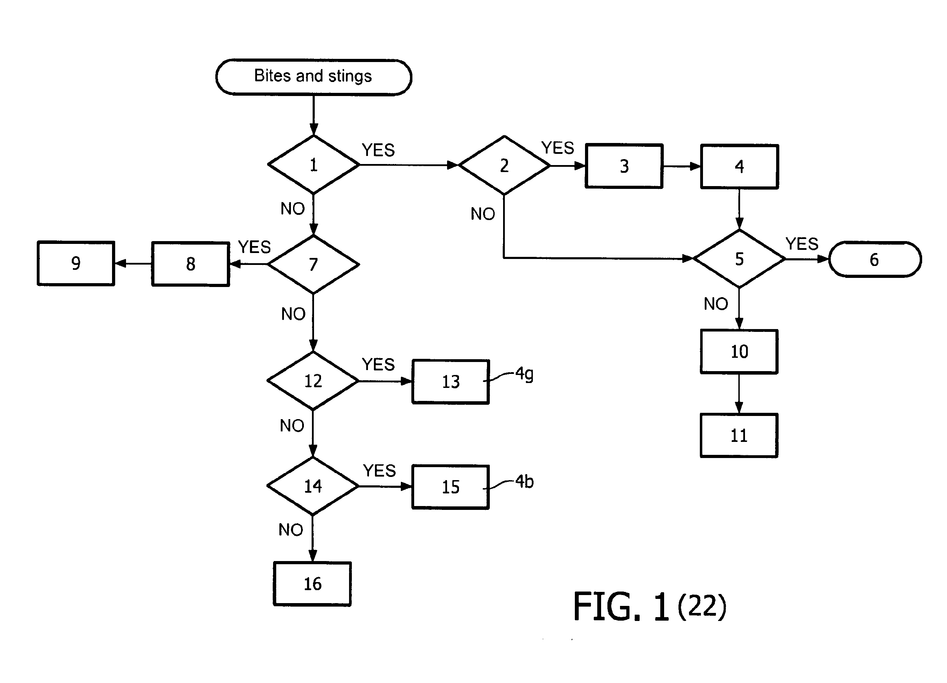

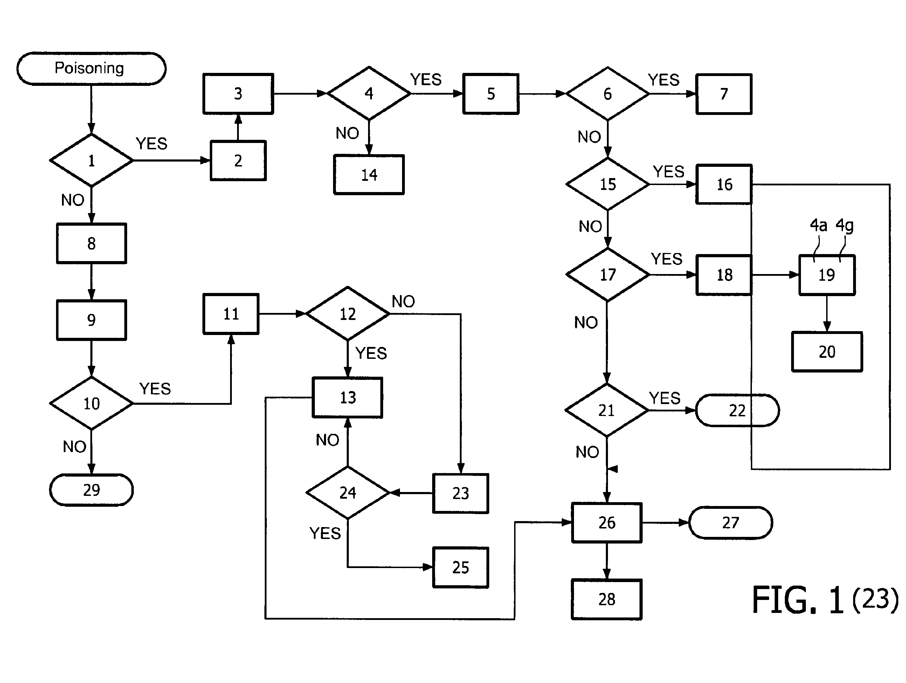

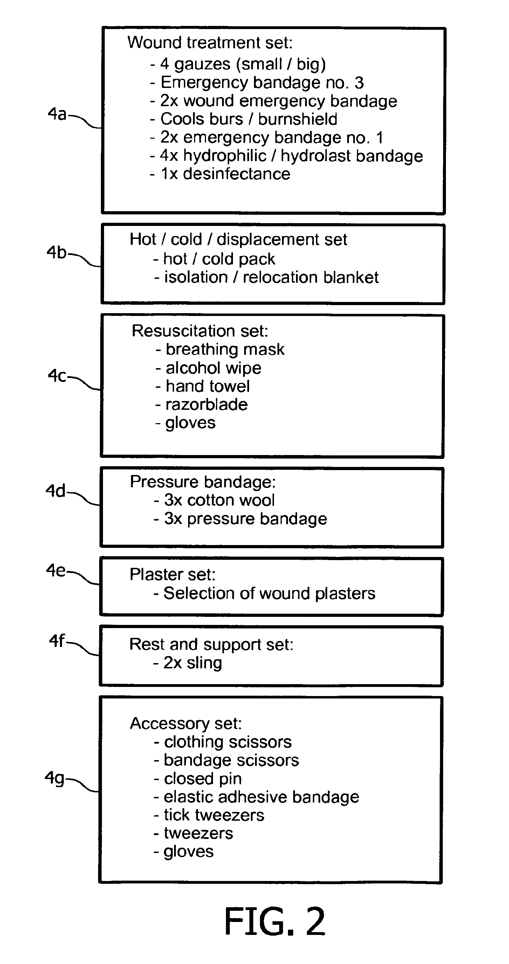

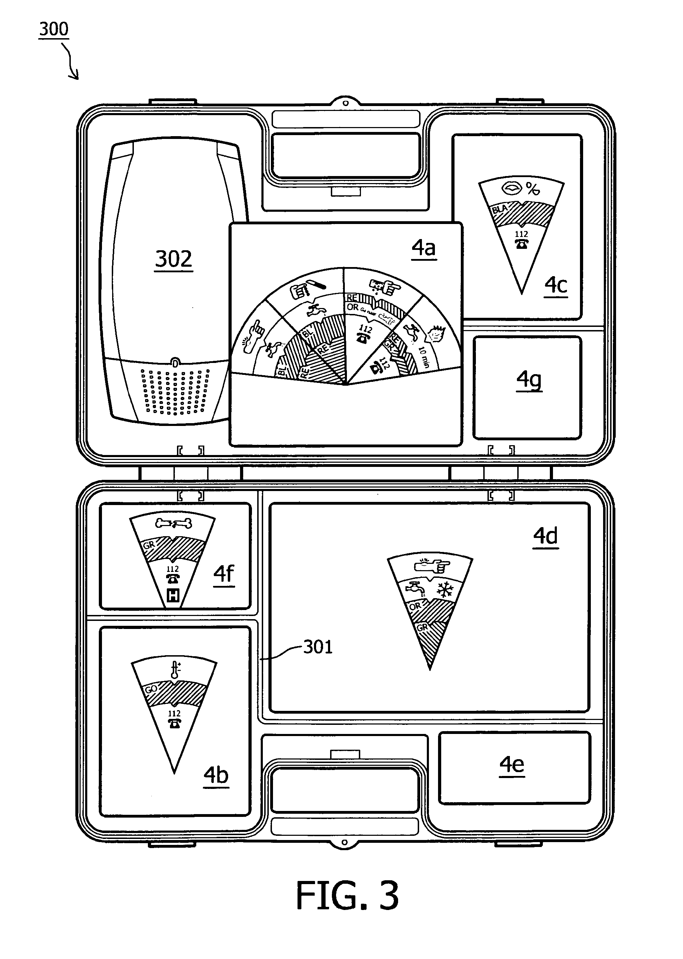

Figure 1 shows a decision tree 100 for following the method in accordance with this invention. A number of symbols are used in the decision tree. An oval 1 indicates an action to be taken with regard to going through the tree or with regard to a device for implementing the method in accordance with this invention, such as a "Safety Mate" or "AED". An arrow 1a may run from an oval. Such an arrow points to the next symbol, which may be a diamond 2 or rectangle 3. A diamond 2 indicates a question that needs to be answered to go through the decision tree. The questions are formulated in such a way that they can be answered with "yes" or "no". Each answer is associated with an arrow, 2a, 2b, which refers to a symbol which indicates a particular action to be taken in the case in question. A rectangle 3 indicates one or more steps to be taken. Arrows 3b and 3a respectively may run from and to a rectangle 3. An arrow 1b pointing to an oval 1' indicates that particular action has to be taken with regard to going through the tree, such as proceeding to an oval referred to. An arrow 1b pointing to an oval 1' may also indicate a particular action with regard to a device for carrying out the method in accordance with this invention. For the actual procedural steps to which the symbols refer, reference is made to the text in the figure for clarity's sake. It should be noted in particular that a number of symbols are indicated by 4a-4g. These symbols comprise references to one of the sets of bandages or injury kits 4a-4g (see figure 2) which are stored in a first-aid kit, indicated in figure 1 by "QRS Kit" (see figure 3). A device for issuing instructions, in particular asking questions from diamond 2 in the figure, is indicated in figure 1 by AED.

Figure 2 shows the content of sets of bandages 4a-4g as used in the decision tree in figure 1. It should be noted in particular that the quantities mentioned should not be considered limitative. It is possible that the sets have different quantities. It is also possible that components from the set of accessories 4g are included in one or more of the other injury kits 4a-4f. The protocols shown do not include a reference to the plaster set 4^{e}. The use of this set is fairly self-evident in the case of minor bleeding injuries. However, it is conceivable that a reference to this set is also included.

Figure 3 shows a first-aid kit 300 equipped with the sets of bandages 4a-4g in figure 2, and also a device 302 for going through the method in accordance with this invention. A partition 301 can be seen in the figure which serves to form compartments inside first-aid kit 300. These compartments are of such a shape and size as to exactly accommodate one or more sets of bandages 4a-4g.

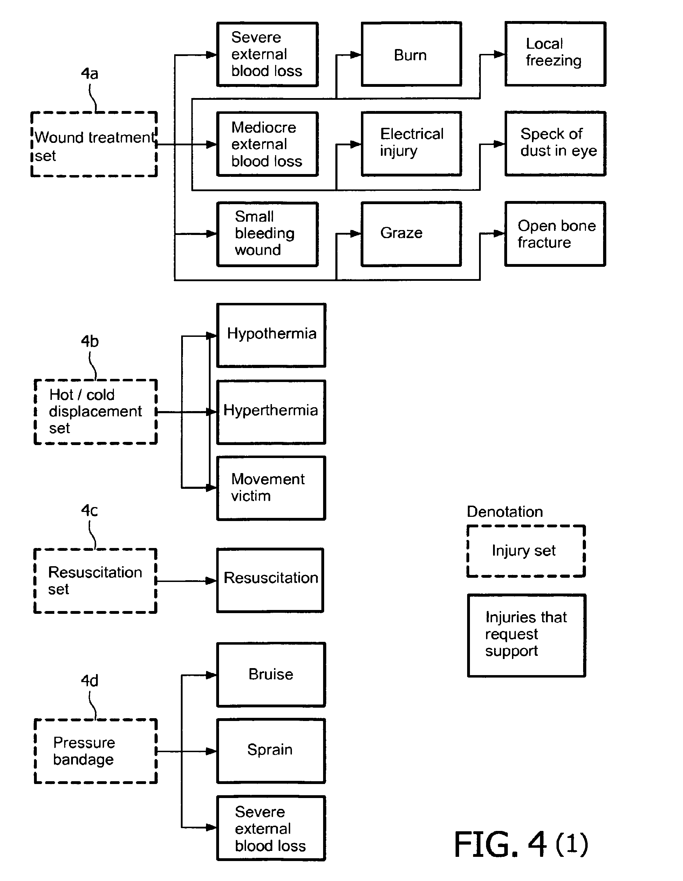

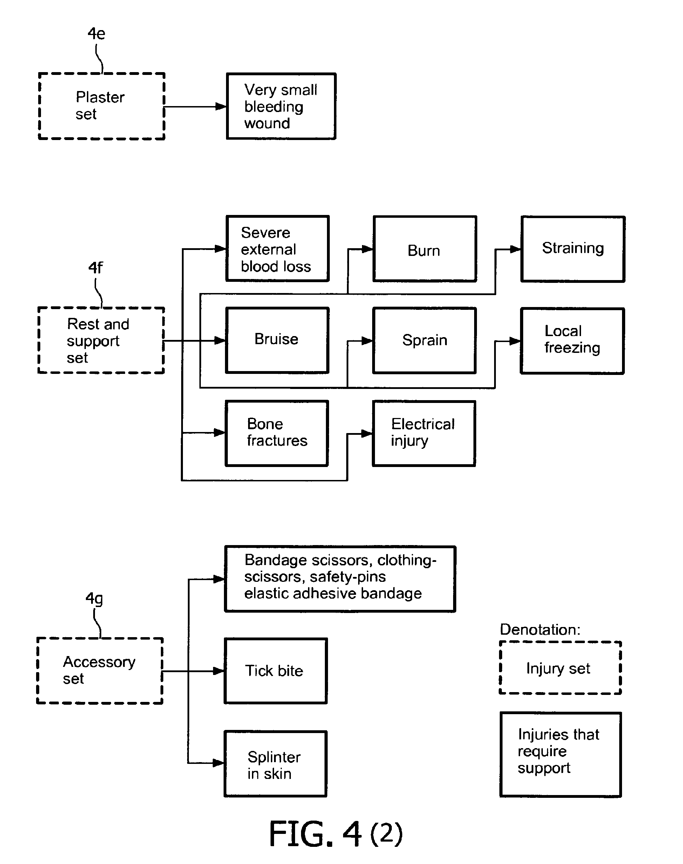

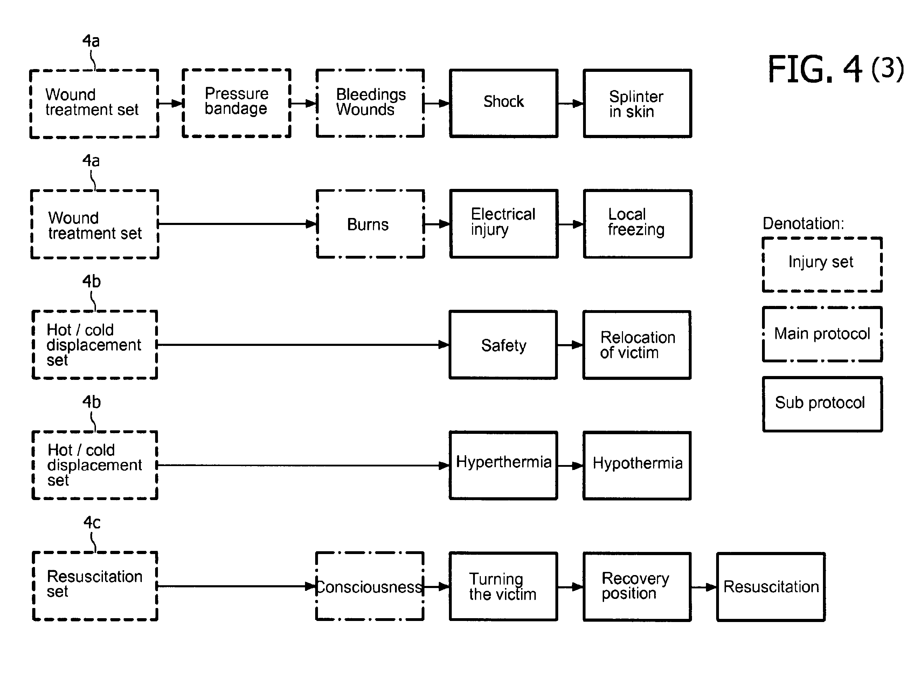

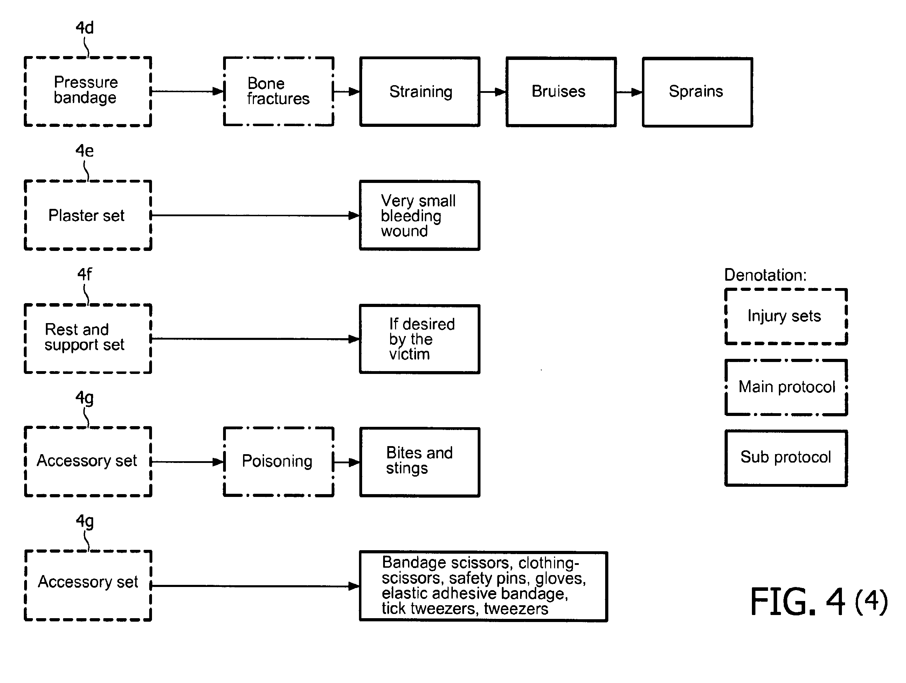

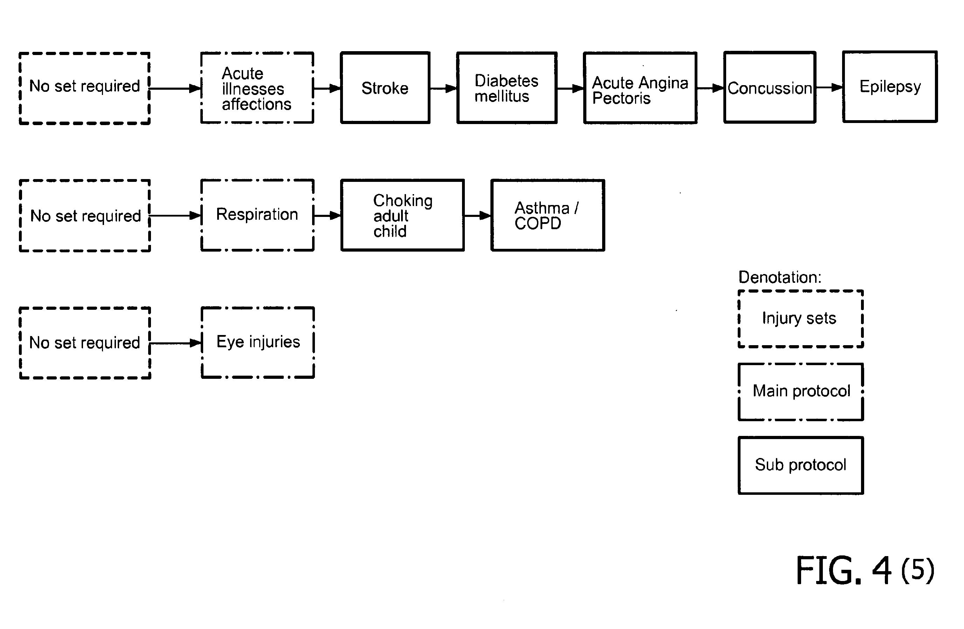

Figure 4 shows an overview of the sets of bandages 4a-4g in figure 2 together with their areas of application. A complete first-aid kit comprises all sets of bandages but it is conceivable that customised first-aid kits or specifically configured devices for going through the protocol are put together. For this purpose, a company may, for instance, be scanned to pinpoint the risks (types of injury), after which the associated protocols can be installed and the remedies adapted accordingly. In this context, a distinction would be made between, for example, companies in the building sector and companies in the chemical sector. A company of the latter type will have a greater need for protocols relating to poisoning after a chemical gas cloud has been emitted.

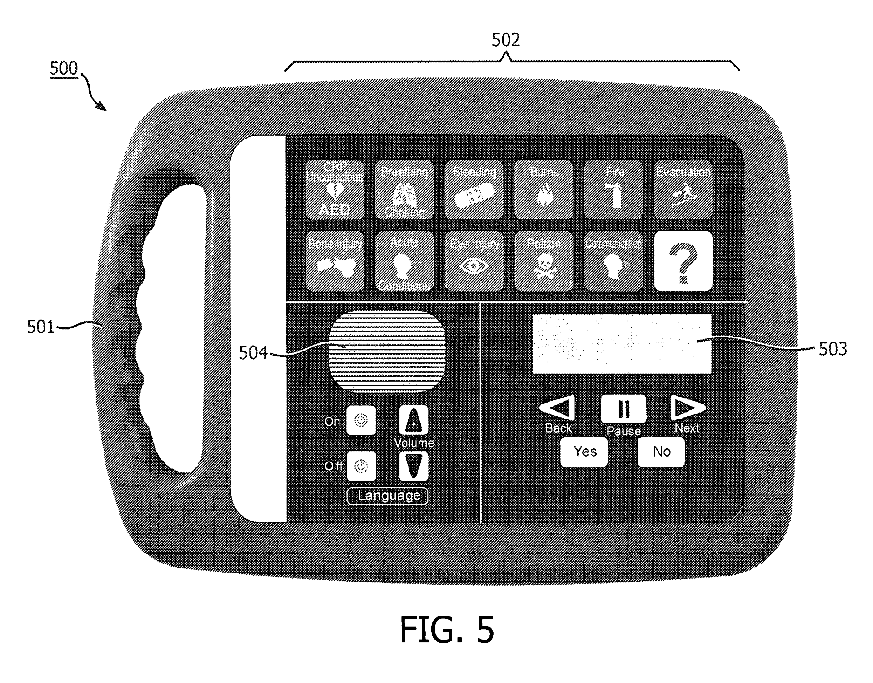

Figure 5 shows a device 500 in accordance with this invention for going through a protocol. The device includes a handle 501, operating components 502, a display 503 and a loudspeaker and microphone 504.

The method in accordance with the invention can also be applied to non-medical protocol-based activities, or training in other fields such as, for instance, evacuation drills for a building. These and other forms of protocol-based action can be controlled using the equipment pertaining thereto.

Apart from the versions shown, which are not limitative, there are many versions possible which are all covered by the scope of protection of the following claims.

It should be noted in particular that a device in accordance with this invention can be used to go through a decision tree in which protocols for first aid are combined with those for fire and evacuation, thus making it possible for the device to support a comprehensive in-house emergency service. For this purpose, terminology for training or retraining and current use can be fully harmonised during emergencies.

More generally, the device in accordance with the invention can be used to go through protocols in general, and in particular in the case of a manual or a checklist that has to be gone through meticulously and in a controlled manner. In particular, this includes going through interactive voice-controlled protocols.

### Text referring to the figures

### Figure 1-1

### START

### Open lid

1: Is this an acute emergency? 2: Have someone call 112 or do it yourself if you are alone. Describe the situation. 3: Is the victim lying safely? 4: If Safety Mate asks a question, press the YES or NO button. 5: Go to the chosen injury by pressing the correct button. 6: If no choice is made, go to Turn Safety Mate On. 7: Is it safe for you to approach the victim? 8: Move the victim to a safe place. Say: I am moving you to a safe place (ONLY IF POSSIBLE). Go to the victim. 9: Kneel on the left side at the victim's shoulder. Place your right foot behind the victim's head. Put your right hand under the victim's neck and put your fingers under his right armpit. From the back, put your left hand under the victim's left armpit. Place the victim in a sitting position.Slide your arms under the victim's armpits. Place one of the victim's forearms horizontally in front of his chest. Place your hands with closed fingers and thumbs over this forearm. Assume a crouching position as near as possible to the victim. Lift the victim up by straightening your legs. Drag the victim away from the danger zone. Carefully lay the victim down. 10: Wait for professional help from a safe distance.

### Figure 1-2

### Turn Safety Mate On

1: Is the victim conscious? Can the victim open his eyes or can he answer your questions? 2: Is his breathing normal? 3: Can you see any blood? 4: Go to Bleeding. 5: Is there a lot of bleeding over a short period? 6: Press your hands on the wound. Ask someone to fetch the QRS Kit. From the wound care kit, take a bandage with red text or a clean cloth. Place this on the wound. 7: Go to Consciousness 1. 8: Does the victim have pain in his chest? 9: Go to Angina Pectoris 10: Go to Choking/Breathing. 11: Does the victim have several of the following symptoms: pale, cold, clammy, rapid shallow pulse and breathing, flaccid muscles, thirst, nausea, restlessness, dry mouth and lips? 12: Go to Shock. 13: Are there any burns? 14: Go to Bums. 15: Do you suspect any back or head injuries? 16: Go to Bone Fractures. 17: Do you suspect any bone fractures? 18: Does the victim have pain in his eyes? 19: Go to Eye Injuries. 20: Do you think this may be a case of poisoning? 21: Go to Poisoning. 22: Is there acute illness or another acute condition? 23: Go to Acute Illnesses/Conditions.

### Figure 1-3

### Turn Safety Mate 1 On

1: Is breathing normal? 2: Go to Breathing/Choking. 3: Is there any blood? 4: Go to Bleeding. 5: Does the victim have several of the following symptoms: pale, cold, clammy, rapid shallow pulse and breathing, flaccid muscles, thirst, nausea, restlessness, dry mouth and lips? 6: You cannot relieve shock. Have someone call 112. Do it yourself if you are alone. Leave the victim lying where he is. Ask someone to fetch the QRS Kit. Take out the cool set. Use an insulation blanket to stop the victim from cooling down. Do NOT let the victim drink anything. Check for local injuries. Keep the airways free. Evaluate any local injuries. 7: For other information, press the correct button on Safety Mate. 8: Are there any burns? 9: Go to Burns. 10: Do you suspect any back or head injuries? 11: Go to Bone Fractures. 12: Do you suspect any broken bones? 13: Does the victim have pain in his eyes? 14: Go to Eye Injuries. 15: Do you think this may be a case of poisoning? 16: Go to Poisoning. 17: Is there acute illness or another acute condition? 18: Go to Acute Illnesses/Conditions.

### Figure 1-4

### Safety

1: Is it safe for you to approach the victim? 2: Move the victim to a safe place. Say: I am moving you to a safe place (ONLY IF POSSIBLE). Go to the victim. 3: Kneel on the left side at the victim's shoulder. Place your right foot behind the victim's head. Put your right hand under the victim's neck and put your fingers under his right armpit. From the back, put your left hand under the victim's left armpit. Place the victim in a sitting position. Slide your arms under the victim's armpits. Place one of the victim's forearms horizontally in front of his chest. Place your hands with closed fingers and thumbs over this forearm. Assume a crouching position as near as possible to the victim. Lift the victim up by straightening your legs. Drag the victim away from the danger zone. Carefully lay the victim down. Go to Consciousness 1 5: Wait for professional help from a safe distance.

### Figure 1-5

### Shock

1: Watch out for danger. Signs of shock are: pale, cold, clammy, rapid shallow pulse and breathing, flaccid muscles, thirst, nausea, restlessness, dry mouth and lips. 2: Is the victim conscious? 3: Is there any major bleeding? 4: Press your hands on the wound. Ask someone to fetch the QRS Kit. From the wound care kit, take a bandage with red text or a clean cloth. Place this on the wound. 5: Go to Bleeding. 6: Go to Consciousness 1. 7: You cannot relieve shock. Have someone call 112. Do it yourself if you are alone. Leave the victim lying where he is. Ask someone to fetch the QRS Kit. Take out the cool set. Use an insulation blanket to stop the victim from cooling down. Do NOT let the victim drink anything. Check for local injuries. Keep the airways free. Evaluate any local injuries. 8: For other information, press the correct button on Safety Mate.

### Figure 1-6

### ASTHMA/COPD

1: Is the victim conscious? 2: You do not hear normal breathing. The victim is clearly having difficulty breathing. 3: Has the victim choked on something? 4: Is the victim still able to talk, cough or breathe? 5: Encourage the victim to keep coughing. Take no further action. Stay with the victim until he is breathing normally again. 6: Go to Consciousness 1. 7: Does the victim suffer from asthma or COPD? 8: Loosen victim to get into a comfortable any tight clothing around the victim's neck. Help theposition. Help the victim to rest. Help the victim to take his own medicine. Do NOT give the victim anything to eat or drink. 9: If the victim does not have any medicine. If the victim does not improve after taking his own medicine. If the victim deteriorates. If the victim turns blue. Have someone call 112 or do it yourself if you are alone. Describe the situation. Monitor the victim's breathing. 10: Is the victim still conscious? 11: Stay with the victim. 12: Have someone call 112 or do it yourself if you are alone. Describe the situation. 13: Go to Consciousness. 14: Go and stand to one side somewhat behind the victim. Support his chest with one hand. Bend the victim slightly forwards. Hit him firmly five times between the shoulder blades with the heel of your other hand 1, 2, 3, 4, 5. Stop immediately as soon as the airways are free again. 15: Is he breathing normally? 16: Do not take any further action. Victims who continue coughing, have difficulty swallowing or have the feeling that there is still something stuck in their throat should see a doctor. 17: Go and stand behind the victim. Clasp your hands together around the upper part of the victim's abdomen. Bend the victim forward. Make a fist and place it between the victim's navel and the lower tip of the breastbone. Grab your fist with your other hand. With a quick jerk, thrust both hands upward towards you. Do this five times 1, 2, 3, 4, 5. 18: Is the victim breathing normally? 19: Call a doctor. 20: Is the victim still conscious? 21: Have someone call 112 or do it yourself if you are alone. Describe the situation. 22: Ask someone to fetch the QRS Kit and the AED if available. Switch the AED on and follow the instructions. Take the resuscitation set out of the QRS Kit. Dry the chest. Use the clothing scissors if necessary. Use the resuscitation mask. 23: Start resuscitation until the AED is available. Kneel beside the victim. Place the heel of your hand in the middle of the victim's breastbone. Place the heel of your other hand on top. Keep your elbows straight. Try not to press down on the ribs. Press the chest in 4 to 5 cm to the rhythm of the beeps. 24: Go to Resuscitation.

### Figure 1-7

### Consciousness

1: Is the victim lying safely? 2: Approach the victim from the front. Speak to the victim. Carefully shake him by the shoulders. Ask "Are you OK?" 3: Does the victim respond? 4: Leave the victim lying in the position in which you found him. 5: Does the victim react normally? 6: Does the victim have difficulty breathing? 7: Does the victim have asthma/COPD? 8: Loosen any tight clothing around the victim's neck. Help the victim to get into a comfortable position. Help the victim to rest. Help the victim to take his own medicine. Do NOT give the victim anything to eat or drink. If the victim does not have his own medicine. If the victim does not have any medicine. If the victim does not improve after taking his own medicine. If the victim deteriorates. If the victim turns blue. Have someone call 112 or do it yourself if you are alone. Describe the situation. Monitor the victim's breathing. 9: Is the victim still conscious? 10: Stay with the victim. 11: Go to Safety. 12: Go to Acute Conditions 1. 13: Go to Turn Safety Mate On. 14: Have someone call 112 or do it yourself if you are alone. Describe the situation. 15: Is the victim still conscious? 16: Loosen any tight clothing around the victim's neck. Help the victim to get into a comfortable position. Help the victim to rest. Help the victim to take his own medicine. Do not give the victim anything to eat or drink. 17: Call for help. 18: Is the victim lying on his back? 19: Check his breathing. Position yourself so that your cheek and ear are above the mouth and nose of the victim. Lift his chin and head up. Check if his chest and upper abdomen are moving. Listen whether the victim is breathing. Feel if there is any warm air. 20: Is the victim breathing normally? 21: Go to Recovery Position. 22: Have someone call 112 or do it yourself if you are alone. Describe the situation. 23: Ask someone to fetch the QRS Kit and the AED if available. Switch the AED on and follow the instructions. Take the resuscitation set out of the QRS Kit. Dry the chest. Use the clothing scissors if necessary. Use the resuscitation mask. 24: Start resuscitation until the AED is available. Kneel beside the victim. Place the heel of your hand in the middle of the victim's breastbone. Place the heel of your other hand on top. Keep your elbows straight. Try not to press down on the ribs. Press the chest in 4 to 5 cm to the rhythm of the beeps. 25: Go to Resuscitation. 26: Be aware of possible head, neck or back injuries if the victim was involved in an accident, such as a road accident, a fall or if there are serious head wounds. Do you suspect any back injury? 27: Leave the victim lying the way you found him. Check his breathing above his mouth and nose. Lift his chin and head up. Check if his chest and upper abdomen are moving. Listen whether the victim is breathing. Feel if there is any warm air. 28: Is the victim breathing normally? 29: Go to Recovery Position. 30: Have someone call 112 or do it yourself if are alone. Describe the situation. 31: Maintain the head, neck and trunk in a single line if possible if you are putting the victim on his back. 32: Ask someone to fetch the QRS Kit and the AED if available. Switch on the AED and follow the instructions. Take the resuscitation set out of the QRS Kit. Dry the chest. Use the clothing scissors if necessary. Use the resuscitation mask. 33: Start resuscitation until the AED is available. Kneel beside the victim. Place the heel of your hand in the middle of the victim's breastbone. Place the heel of your other hand on top. Keep your elbows straight. Try not to press down on the ribs. Press the chest in 4 to 5 cm to the rhythm of the beeps. 34: Go to Resuscitation. 35: Kneel beside the victim at the back of his head. Carefully place the victim's arm that is nearest to you beside his head, pointing upwards. Let the hand of that arm rest flat on the floor with the back of the hand upwards (pause). Place the victim's arm that is furthest away from you alongside his body. Let the hand of that arm rest flat on the floor with the palm facing upwards (pause). Place the leg that is furthest away from you over the other leg (pause). Grasp the victim by the hip and shoulder. Also grasp the arm that is alongside the hip (pause). Turn the victim towards you until he is lying on his side (pause). Let go of the shoulder, place your hand under the victim's head. Turn the victim until he is lying on his back.

### Figure 1-8

### Consciousness 1

1: Approach the victim from the front. Speak to the victim. Say: Can you hear me? What happened? 2: Does the victim respond? 3: Leave the victim lying as you found him. 4: Does the victim react normally? 5: Does the victim have difficulty breathing? 6: Does the victim have asthma or COPD? 7: Loosen any tight clothing around the victim's neck. Help the victim to get into a comfortable position. Help the victim to rest. Help the victim to take his own medicine. Do NOT give the victim anything to eat or drink. If the victim does not have his own medicine. If the victim does not have any medicine.If the victim does not improve after taking his own medicine. If the victim deteriorates. If the victim turns blue. Have someone call 112 or do it yourself if you are alone. Describe the situation. Monitor the victim's breathing. 8: Is the victim still conscious? 9: Stay with the victim. 10: Go to Acute Conditions. 11: Go to TURN SAFETY MATE 1 ON 12: Have someone call 112 or do it yourself if you are alone. Describe the situation. 13: Is the victim still conscious? 14: Loosen any tight clothing around the victim's neck. Help the victim to get into a comfortable position. Help the victim to rest. Help the victim to take his own medicine. Do NOT give the victim anything to eat or drink. 15: Call for help. 16: Is the victim lying on his back? 17: Check his breathing. Position yourself so that your cheek and ear are above the mouth and nose of the victim. Lift his chin and head up. Check if his chest and upper abdomen are moving. Listen whether the victim is breathing. Feel if there is any warm air. 18: Is the victim breathing normally? 19: Go to Recovery Position. 20: Have someone call 112 or do it yourself if you are alone. Describe the situation. 21: Ask someone to fetch the QRS Kit and the AED if available. Switch the AED on and follow the instructions. Take the resuscitation set out of the QRS Kit. Dry the chest. Use the clothing scissors if necessary. Use the resuscitation mask. 22: Start resuscitation until the AED is available. Kneel beside the victim. Place the heel of your hand in the middle of the victim's breastbone. Place the heel of your other hand on top. Keep your elbows stretched. Try not to press down on the ribs. Press the chest in 4 to 5 cm to the rhythm of the beeps. 23: Go to Resuscitation. 24: Be aware of possible head, neck or back injuries if the victim was involved in an accident, such as a road accident, a fall or if there are serious head wounds. Do you suspect any back injury? 25: Leave the victim lying as you found him. Check the victim's breathing above his mouth and nose Lift his chin and head up. Check if the chest and upper abdomen are moving. Listen whether the victim is breathing. Feel if there is any warm air. 26: Is the victim breathing normally? 27: Go to Recovery Position. 28: Kneel beside the victim at the back of his head. Carefully place the victim's arm that is nearest to you beside his head, pointing upwards. Let the hand of that arm rest flat on the floor with the back of the hand upwards (pause). Place the victim's arm that is furthest away from you along side the body. Let the hand of that arm rest flat on the floor with the palm facing upwards (pause). Place the leg that is furthest away from you over the other leg (pause). Grasp the victim by the hip and shoulder. Also grasp the arm that is alongside the hip (pause) Turn the victim towards you until he is lying on his side (pause). Let go of the shoulder, place your hand under the victim's head. Turn the victim until he is lying on his back. 29: Have someone call 112 or do it yourself if you are alone. Describe the situation. 30: Maintain the head, neck and trunk in a single line if possible if you are putting the victim on his back. 31: Ask someone to fetch the QRS Kit and the AED if available. Switch the AED on and follow the instructions. Take the resuscitation set out of the QRS Kit. Dry the chest. Use the clothing scissors if necessary. Use the resuscitation mask. 32: Start resuscitation until the AED is available. Kneel beside the victim. Place the heel of your hand in the middle of the victim's breastbone. Place the heel of your other hand on top. Keep your elbows straight. Try not to press down on the ribs. Press the chest in 4 to 5 cm to the rhythm of the beeps. 33: Go to Resuscitation.

### Figure 1-9

### Recovery Position

1: Be aware of possible head, neck or back injuries if the victim was involved in an accident involving a severe impact on the body. As in a road accident or a fall or if there is a visible head injury. 2: Could there be any head, neck or back injury or injury to the chest? 3: Maintain the head, neck and trunk in a single line if possible if you are putting the victim in the recovery position. 4: Remove the victim's glasses. Kneel beside the victim. Make sure that the victim's legs are straight (pause). Place the victim's arm nearest to you at right angles to his body (pause). Carefully place his other arm over his chest with the elbow bent. Keep the back of that hand against the victim's cheek on your side (pause). With your other hand, grasp the leg that is furthest away from you just above the knee and pull it up so it is bent (pause). Keep the victim's hand pressed against his cheek. Pull on the furthest leg (pause). Tilt the victim towards you. Bend the victim's uppermost leg so that the knee touches the ground. Make sure that the victim's mouth is turned towards the ground and his head is lifted up. 5: Have someone call 112 or do it yourself if you are alone. Describe the situation. 6: Check the victim's breathing every minute. 7: Has the victim stopped breathing? 8: Turn the victim on his back. 9: Ask someone to fetch the QRS Kit and the AED if available. Switch the AED on and follow the instructions. Take the resuscitation set out of the QRS Kit. Dry the chest. Use the clothing scissors if necessary. Use the resuscitation mask. Start resuscitation until the AED is available. Kneel beside the victim. Place the heel of your hand in the middle of the victim's breastbone. Place the heel of your other hand on top. Keep your elbows straight. Try not to press down on the ribs. Press the chest in 4 to 5 cm to the rhythm of the beeps. 11: Go to Resuscitation. 12: For other information, press the correct button on the Safety Mate. 13: Stay with the victim until professional help arrives.

### Figure 1-10

### Burns

1: Is the victim on fire? 2: Extinguish the flames. Ask someone to fetch the fire blanket. Tell the victim to lie down. Use the fire blanket or water. Have someone call 112 or do it yourself if you are alone. Describe the situation. 3: Check if the victim remains conscious. Monitor his breathing. If he is not breathing, press the Consciousness button on Safety Mate. 4: Is it caused by chemical burns? 5: Think of your own safety! Have someone call 112 or do it yourself if you are alone. Describe the situation. 6: Ask someone to fetch the QRS Kit. Take out the set of accessories. Put on gloves. Remove clothing and jewellery if they are NOT stuck to the victim's skin. Rinse the victim for at least 30 minutes. Do not use cold water. Get the wound care kit. Cover wounds with gauze with red text.. 7: Stay with the victim. 8: Cool for 15-20 minutes with lukewarm, running tap water. Remove clothing and jewellery if they are NOT stuck to the victim's skin. Do not pierce blisters. Ask someone to fetch the QRS Kit. 9: Is more than 10% of the body burnt? 10: Have someone call 112 or do it yourself if you are alone. Describe the situation. 11: Go to Shock. 12: Is the face burnt? 13: See if there are any problems with breathing. 14: Have someone call 112 or do it yourself if you are alone. Describe the situation. 15: Are the bums spread over the whole body? 16: Have someone call 112 or do it yourself if you are alone. Describe the situation. 17: Is the skin black? 18: Get the wound care kit. Cover bums with gauze with red text. Position the gauze loosely and secure it with a roll of bandage with green text. Do NOT rub anything onto the burn. 19: Consult a doctor. 20: Is the skin red, swollen, painful and blistered? 21: Do not take any further action.

### Figure 1-11

### Breathing/Choking

1: Is the victim conscious? 2: Has the victim choked on something? 3: Can the victim still talk, cough or breathe? 4: Encourage the victim to keep coughing. Do not take any further action. Stay with the victim until he is breathing normally again. 5: Go to Consciousness. 6: Go to Consciousness. 7: Go and stand to one side somewhat behind the victim. Support his chest with one hand. Bend the victim slightly forwards. Hit him firmly five times between the shoulder blades with the heel of your other hand. 1, 2, 3, 4, 5. Stop immediately as soon as the airways are free again. 8: Are the airways free? 9: Do not take any further action. Victims who continue coughing, have difficulty swallowing or have the feeling that there is something stuck in their throat should see a doctor. 10: Go and stand behind the victim. Clasp your hands together around the upper part of the victim's abdomen. Bend the victim forward. Make a fist and place it between the victim's navel and the lower tip of the breastbone. Grab your fist with your other hand. With a quick jerk, thrust both hands upward towards you. Do this five times 1, 2, 3, 4, 5. 11: Are the airways free? 12: Call a doctor. 13: Is the victim still conscious? 14: Have someone call 112 or do it yourself if you are alone. Describe the situation. 15: Ask someone to fetch the QRS Kit and the AED if available. Switch on the AED and follow the instructions. Take the resuscitation set out of the QRS Kit. Dry the chest. Use the clothing scissors if necessary. Use the resuscitation mask. 16: Start resuscitation until the AED is available. Kneel beside the victim. Place the heel of your hand in the middle of the victim's breastbone. Place the heel of your other hand on top. Keep your elbows straight. Try not to press down on the ribs. Press the chest in 4 to 5 cm to the rhythm of the beeps. 17: Go to Resuscitation.

### Figure 1-12

### Baby choking

1: Corresponds to EFAM Child/Baby in version 2.

### Figure 1-13

### Resuscitation

1: Ready, start. 1, 2, 3, 4... Compress chest 30 times, administer respiration twice and repeat. Be prepared to tilt the head. Lift the chin up. Close the soft part of the nose with your thumb and forefinger. Administer respiration twice ....28, 29, 30. 2: Blow. Blow. Again. 3: Ready, start. 1, 2, 3, 4... Keep your arms straight and your shoulders directly above your hands. Allow the victim's chest to rise properly. 28, 29, 30. 4: Blow. Blow. Again. 5: Ready, start. 1, 2, 3, 4... If the victim vomits, turn him immediately on his side towards you. Keep the airways free..... 28, 29, 30. 6: Blow. Blow. Again. 7: Ready, start. 1, 2, 3... Continue with resuscitation until the AED is available. Or if you see clear signs of life. Or if you are exhausted and someone else takes over....28, 29, 30. 8: Blow. Blow. Again. 9: Ready, start. 1, 2, 3... Continue until the AED gives you instructions or until the ambulance service takes over. ... 28, 29, 30. 10: Blow. Blow. Again. 11: Ready, start. 1, 2, 3... 28, 29, 30.

### Figure 1-14

### Eye injuries

1: The victim feels pain in one or both eyes. The eye may be red. The eyes may tear. The victim keeps the painful eye tightly shut. The victim's vision is reduced. 2: Ask someone to fetch the QRS Kit. 3: If you see something in the eye, do NOT try to take it out. 4: Is there blood in the eye? Is the pupil abnormal? 5: Make sure that the victim does not rub his eye. Ask the victim not to move his eyes. Let the victim lie down or help him into a half-sitting position. Cover both eyes with an eye shield. Do not use any gauze or cloth. Take the victim to a doctor. 6: Is the eye burnt? 7: Let the victim lie down. Keep the eye open or have it kept open. Rinse out the eye with an eye douche or gently running water. Take the victim to a doctor. 8: Have chemical substances got into the eye? 9: Let the victim lie down. Keep the eye open or have it kept open. Rinse out the eye with an eye douche or gently running water. Whilst doing so, keep the victim's head inclined. Rinse from the side of the nose towards the side of the face. Attention! Do not allow any dirty eye water to enter the other eye. Have someone call 112 or do it yourself if you are alone. Describe the situation. 10: Did the victim receive a blow to the eye with a blunt object? 11: Cool the area around the eye. Take the victim to a doctor if he is confused. 12: Did the victim receive a bright light in his eye? Such as bright sunlight or welding equipment? 13: Take the victim to a doctor. 14: Does the victim have a loose particle of dirt in his eye? 15: Can you see the particle? 16: With your thumb and forefinger, pull the eyelids carefully apart. Remove the particle from the eye with the tip of a clean gauze or clean handkerchief. Only if the particle of dirt is in the white of the eye. Move the particle to the nearest corner of the eye. Or wash the eye out with water. Rinse from the side of the nose towards the side of the face. 17: Let the victim look up. Pull down the lower eyelid. Let the victim look down. Pull up the upper eyelid. Get the wound care kit. Remove the particle from the eye with the tip of a gauze with red text or with a clean handkerchief. Consult a doctor if the particle is on the coloured part of the eye. Consult a doctor if you cannot get the particle out. 18: If the pain persists, consult a doctor. Do the same if the victim has reduced vision. 19: Do nothing, the particle is probably already gone. If the pain persists, consult a doctor.

### Figure 1-15

### Bleeding/wounds

1: Is the victim conscious? 2: Let the victim lie down or sit up. Have the victim press on the wound. Ask someone to fetch the QRS Kit. 3: Is there a lot of bleeding over a short period? 4: Have someone call 112 or do it yourself if you are alone. Describe the situation. 5: Get the set of accessories. Put on gloves or use a plastic bag if available to cover your hands. 6: Do not remove anything from the wound. 7: Press on the wound, using your hands, gauze with red text, or a clean cloth. 8: Get the pressure bandages. Use pressure bandage according to the colour guide. 9: Apply the bandage sufficiently tightly so that bleeding stops without constricting the blood vessel. 10: Has the bleeding stopped? 11: Has the part of the body beyond the wound turned blue? 12: Loosen the bandage a bit but do not take it off. 13: Keep pressing the wound closed until the ambulance service can take over. 14: Go to Consciousness. 15: Press a little harder on the wound or put a second bandage around the first one. Do not remove the first bandage. 16: Is the wound very dirty? Was it caused by a bite? Is something sticking out of the wound? Is the graze/cut bigger than the palm of your hand? Are bones, muscles or other tissue visible? Does the wound affect the face, eyes or genitals? 17: Get the set of accessories. Put on gloves. Get the wound care kit. Try to stabilise whatever is sticking out of the wound with two rolls of bandage with green text. Let the victim remove any jewellery himself. Do not clean the wound. Cover the wound with a gauze with red text. Take the victim to a doctor. 18: Does the victim have a nosebleed? 19: Let the victim sit up with his head tilted forwards. Let him blow his nose. Press the nostrils against the septum. Continue this for 10 minutes. If the bleeding persists, consult a doctor. Consult a doctor if the bleeding was caused by a blow to the nose. 20: Get the set of accessories. Put on gloves. Let the victim remove any jewellery himself. Rinse the wound under the tap. Dry the wound. Cover the wound with sterile gauze with red text according to the colour guide. If in doubt, consult a doctor. 21: Is the victim still conscious? 22: Do not move the victim. Get the cool set. Use an insulation blanket to stop the victim from cooling down. Do not let the victim eat or drink anything. Check for any other injuries. Monitor the victim's breathing. 23: For other information, press the correct button on Safety Mate. Wash your hands. 24: Go to Consciousness 1.

### Figure 1-16

### Bone fractures

1: Is the victim conscious? 2: Does the victim have any neck or back pain or was he involved in an accident involving a severe impact to the body? Such as a traffic accident or fall. 3: The victim may feel pins and needles. The victim may have signs of paralysis in his arms and legs. 4: Keep the victim calm and tell him not to move. Have someone call 112 or do it yourself if you are alone. Describe the situation. Immobilise the victim but only if he agrees. If the victim is restless or agitated, do not hold his head and neck against his will. 5: Stay with the victim. 6: Go to Consciousness 1. 7: Does the victim have pain in the ribs? Does it hurt when he breathes? Is his breathing shallow? 8: Place the victim in a half-sitting position. Support the victim. 9: Have someone call 112 or do it yourself if you are alone. Describe the situation. 10: Does the victim feel pain in an arm or leg? Is there any swelling where he feels the pain? 11: Is the arm or leg in an abnormal position? 12: Have someone call 112 or do it yourself if you are alone. Describe the situation. 13: Try NOT to change the position of the part of the body affected. Ask someone to fetch the QRS Kit. Cool the injury using the cool set for not more than 20 minutes. Place a cloth between the ice and the skin. 14: Do not touch the wound if medical help is readily available. Tell the victim not to stand on a damaged lower leg. If there is injury to the upper leg, ask the victim to hold his arm against his body. Ask someone to fetch the QRS Kit. If there is extensive bleeding at the site of the fracture, press directly on the wound and get the wound care kit. Get some gauze with red text. Get the pressure bandage set. Apply it according to the colour guide. 15: Go to Switch Safety Mate On. 16: If you are not sure of the situation. Have someone call 112 or do it yourself if you are alone. Describe the situation. 17: Ask someone to fetch the QRS Kit. If there is extensive bleeding at the site of the fracture, press directly on the wound and get the wound care kit. Get some gauze with red text. Get the pressure bandage set. Apply it according to the colour guide. 18: Is it an arm? 19: Ask someone to fetch the QRS Kit. Support the arm and rest it in the position where you found it using the rest and support set. 20: Call a doctor. Stay with the victim. 21: For other information. Press on the appropriate button on Safety Mate. 22: For fractures of the pelvis, upper leg, knee, lower leg, foot - do not take any action.

### Figure 1-17

### Acute Illnesses

1: Can the victim tell you what happened? 2: Does the victim have difficulty breathing? 3: Go to Asthma/COPD. 4: Ask the victim to show you his teeth. Ask the victim to turn his hands with the palms facing upwards and to lift up both arms. Ask the victim to say a simple sentence. 5: Can the victim carry out all these actions? 6: Let the victim rest and not make any effort. Help the victim to get into a comfortable position. 7: Have someone call 112 or do it yourself if you are alone. Describe the situation. 8: Does the victim feel pain in his chest? 9: Go to ANGINA PECTORIS. 10: Go to Turn Safety Mate 1 On. 11: Does the victim make uncoordinated, violently jerking movements? 12: Try to prevent injury. Do not hinder any movements. Do not put anything in his mouth. 13: Does the fit stop after a few minutes? 14: Did the victim sustain injury during the fit? 15: Have someone call 112 or do it yourself if you are alone. Describe the situation. 16: Did the victim receive a hard knock to the head? 17: Go to Concussion. 18: Is this the first time a fit has occurred? 19: Ask the victim what to do. 20: Does the victim know what to do? 21: Do what he says. 22: Does the victim have several of the following symptoms: sweating, pallor, unclear speech, yawning, mood swings such as stubborn, irritable, sullen, hungry, pins and needles around the mouth, blurred vision, shaking or feeling cold? 23: Let the victim sit up. Give him a lump of sugar. Give him a sandwich or biscuit. Measure his blood sugar level if possible. Stay with the victim as long as necessary. 24: Is the victim getting weaker? 25: Have someone call 112 or do it yourself if you are alone. Describe the situation. 26: Ask the victim what to do. 27: Does the victim know what to do? 28: Do what he says. 29: Go to Turn Safety Mate 1 On.

### Figure 1-18

### Acute Illnesses 1

1: Ask the victim to show you his teeth. Ask the victim to turn his hands with the palms facing upwards and to lift up both arms. Ask the victim to say a simple sentence. 2: Can the victim carry out all these actions? 3: Let the victim rest and not make any effort. Help the victim to get into a comfortable position. 4: Have someone call 112 or do it yourself if you are alone. Describe the situation. 5: Does the victim make uncoordinated, violently jerking movements? 6: Try to prevent injury. Do not hinder any movements. Do not put anything in his mouth. 7: Did the fit stop after a few minutes? 8: Did the victim sustain injury during the fit? 9: Have someone call 112 or do it yourself if you are alone. Describe the situation. Stay with the victim. 10: Is this the first time a fit has occurred? 11: Ask the victim what to do. 12: Does the victim know what to do? 13: Do what he says. 14: Did the victim receive a hard knock to the head? 15: Go to Concussion. 16: Does the victim have several of the following symptoms: sweating, pallor, unclear speech, yawning, mood swings such as stubborn, irritable, sullen, hungry, pins and needles around the mouth, blurred vision, shaking or feeling cold? 17: Let the victim sit up. Give him a lump of sugar. Give him a sandwich or biscuit. Measure his blood sugar level if possible. Stay with the victim as long as necessary. 18: Is the victim getting weaker? 19: Have someone call 112 or do it yourself if you are alone. Describe the situation. 20: Ask the victim what to do. 21: Does the victim know what to do? 22: Do what he says.

### Figure 1-19

### Angina Pectoris

1: Victim has pressing pain in the chest. Victim may have pain radiating to an arm, the shoulder blades, jaws, head. Pain radiating to neck or stomach. This normally goes hand in hand. Breathing problems, sweating, dizziness or fainting. Some patients suffer from nausea and vomiting. 2: Have someone call 112 or do it yourself if you are alone. Describe the situation. 3: Let the victim sit quietly. Help the victim to take his own medicine. 4: If the victim is unconscious, press the Consciousness button.

### Figure 1-20

### Stroke

1: Ask the victim to show you his teeth. Ask the victim to turn his hands with the palms facing upwards and to lift up both arms. Ask the victim to say a simple sentence. If the victim cannot do one or more of these actions, he has probably suffered a stroke. 2: Allow the victim to rest and not make any effort. Help the victim to get into a comfortable position. 3: Have someone call 112 or do it yourself if you are alone. Describe the situation.

### Epilepsy

4: Try to prevent injury. Do not hinder any movements. Do not put anything in the victim's mouth. 5: Did the fit stop after a few minutes? 6: Did the victim sustain serious injury during the fit? 7: Have someone call 112 or do it yourself if you are alone. Describe the situation. In the event of injury, get the QRS Kit. Get the wound care kit. Use the kit according to the colour guide. 8: Is this the first time a fit has occurred? 9: Ask the victim what to do. 10: Does the victim know what to do? 11: Do what he says.

### Figure 1-21

### Concussion

1: Calm the victim down and try to prevent him from moving. 2: Have someone call 112 or do it yourself if you are alone. Describe the situation. 3: Stop the victim from moving. But only if he cooperates. If the victim is restless or irritated, do not hold his head and neck against his will. 4: If the victim loses consciousness, press the CONSCIOUSNESS button. 5: Go to Consciousness 1.

### Figure 1-22

### Bites and stings

1: Has the victim been stung? 2: Has he been stung in the mouth or throat? 3: Immediately call 112. 4: Give him some ice cubes to suck on. Or cold water. This prevents swelling. 5: Are there any: Breathing problems. Extensive swelling or consciousness problems? 6: Go to Consciousness 1. 7: Is it an animal or human bite? 8: Let the victim sit up. Rinse the wound clean under the tap. 9: Consult a doctor. 10: Do not scratch. Pull out the sting with your fingernails. Do NOT use tweezers as the sting may break. Place a cold compress on the sting. 11: Consult a doctor if there are serious symptoms. Call 112 if there is an allergic reaction. Loosen tight clothing / remove jewellery. Help the victim to get into a comfortable, half-sitting position. Do not let the victim eat or drink anything. 12: Is it a tick bite? 13: Fetch the QRS Kit. Get the set of accessories. Remove the tick with tick tweezers. Pull the tick from the skin. Disinfect the wound. Note the date of the tick bite. Consult a doctor if there are any symptoms. 14: Is it a bee or wasp sting? 15: Get the QRS Kit. Get the cool set. Cool the sting with a cold compress. Get the wound care kit. Disinfect the wound. Call 112 if there is an allergic reaction. Consult a doctor if there are any symptoms. 16: For other information, press on the relevant button on Safety Mate.

### Figure 1-23

### Poisoning

1: For cases of Poisoning, If there is more than one victim, the surrounding area may well be dangerous. Do not enter that area. Do you think this is a case of poisoning? 2: If the surrounding area is dangerous, tell everyone to leave. Wait at a safe distance until professional help arrives. Move the victim to a safe place but ONLY IF IT IS POSSIBLE. Say: I am moving you to a safe place. the victim. Go to 3: Kneel on the left side at the victim's shoulder. Place your right foot behind the victim's head. Put your right hand under the victim's neck and put your fingers under his right armpit. From the back, put your left hand under the victim's left armpit. Place the victim in a sitting position. Slide your arms under the victim's armpits. Place one of the victim's forearms horizontally in front of his chest. Place your hands with closed fingers and thumbs over this forearm. Assume a crouching position as near as possible to the victim. Lift the victim up by straightening your legs. Drag the victim away from the danger zone. Carefully lay the victim down. 4: Do you know what type of poison is involved? 5: Find out: the name or description of the poison, how much was eaten or drunk or how much entered the victim's body. Ask about the victim's age and approximate weight. The time that the poison entered the body. Ask how the victim is feeling now. Have someone call 112 or do it yourself if you are alone. Describe the situation. 6: Did the victim eat or drink the poison? 7: Do not allow the victim to vomit. Do not give him anything to eat or drink. This could be harmful. 8: Signs of an allergic reaction are: redness, itching or a rapid, weak pulse. Nausea, vomiting. Or stomach cramps, breathing problems, coughing, panting or dizziness. 9: The tongue, face or neck may swell up and close off the airways. 10: Do you notice any one of these allergic reactions? 11: Have someone call 112 or do it yourself if you are alone. Describe the situation. Look for a medical ID bracelet or necklace. Ask the victim if he has any allergies. 12: Is this the first time that the victim has had an allergic reaction? 13: Keep the victim calm and in a comfortable, half-sitting position. Loosen any tight clothing. Help the victim to get into the best position for breathing. Reassure the victim. Do not give him anything to eat or drink. 14: Think of your own safety! Have someone call 112 or do it yourself if you are alone. Describe the situation. 15: Did the victim breathe in the poison? 16: If you are not sure that the surrounding area is safe, wait for professional help. If the surrounding area is safe, move the victim into the open air. If the victim cannot be moved, open doors and windows. 17: Has the poison ended up on the skin? 18: Think of your own safety! Have someone call 112 or do it yourself if you are alone. Describe the situation. 19: Get the QRS Kit. Take out the set of accessories. Put gloves on. Remove clothing and jewellery if they are NOT stuck to the victim's skin. Rinse the victim for at least 30 minutes. Do not use cold water. Get the wound care kit. Cover any wounds with gauze with red text. Secure it with a roll of bandage with green text. 20: Stay with the victim. 21: Is it a bite or sting from an animal? 22: Go to Bites and Stings. 23: Ask the victim what to do. 24: Does the victim know what to do? 25: Do what he says. 26: If the victim is unconscious, press the Consciousness button. 27: Go to Consciousness 1. 28: For other information, press on the correct button on Safety Mate. 29: Go to Bites and Stings.

## Claims

1. Method for determining a set of bandages appropriate for a particular injury in an emergency situation; comprising:
- providing an interactive voice-controlled audiovisual aid with a decision tree to
○ determine the type of injury on the basis of visual observation of an injured person (symptoms);
○ determine the action to be taken on the basis of the type of injury identified;
○ determine the requisite dressing material on the basis of the action identified; and
○ determine, on the basis of the requisite dressing material thus identified, at least one set of bandages which comprises at least the requisite dressing material.

2. Method in accordance with claim 1, comprising
- on the basis of the type of injury established, determining a control observation to verify the accuracy of the type of injury established.

3. Method in accordance with claim 2, comprising determining the control observation on the basis of a lack of clarity or an inconsistency encountered in going through the decision tree.

4. Method in accordance with one of the above claims, comprising providing an indication, e.g. a letter, icon or colour, of the set of bandages determined.

5. Device for determining a set of bandages appropriate for a particular injury in an emergency situation, comprising audiovisual aids for going interactive voice-controlled through a decision tree to
○ determine the type of injury on the basis of visual observation of an injured person (symptoms);
○ determine the action to be taken on the basis of the type of injury identified;
○ determine the requisite dressing material on the basis of the action identified; and
○ determine, on the basis of the requisite dressing material thus identified, at least one set of bandages which comprises at least the requisite dressing material.

6. Device in accordance with claim 5, comprising:
- a means of audio and/or visual display to present to a user a number of questions from a decision tree; and
- input facilities, e.g. a button or a microphone, to enable a user to enter answers to the questions, for which purpose the decision tree has been loaded or is present in the device.

7. Device according top claim 5 or 6, wherein the audiovisual aid, is a computer or palmtop, configured to guide a care provider interactive voice-controlled through a decision tree to determine a dressing material or a set of bandages appropriate for a particular type of injury.

8. Device in accordance with claim 7, wherein the decision tree, is stored in or on a reusable memory or storage medium such as an SD card.

9. Device in accordance with any of claims 5-8 configured for carrying out at least a training mode and a company mode.

10. Device in accordance with any of claims 9 integrated with a first-aid kit and/or defibrillator.

## Patentansprüche

1. Verfahren zur Bestimmung einer Verbandzusammenstellung für eine bestimmte Verletzung in einer Notsituation, die umfasst:
- die Bereitstellung einer interaktiven sprachgesteuerten audiovisuellen Unterstützung mit einem Entscheidungsbaum zur
○ Bestimmung der Art der Verletzung auf der Grundlage einer visuellen Beobachtung einer verletzten Person (Symptome);
○ Bestimmung der zu treffenden Maßnahmen auf der Grundlage der identifizierten Verletzung;
○ Bestimmung des erforderlichen Verbandmaterials auf der Grundlage der identifizierten Maßnahme; und
○ Bestimmung auf der Grundlage des so identifizierten erforderlichen Verbandmaterials von mindestens einer Verbandzusammenstellung, die zumindest das erforderliche Verbandmaterial umfasst.

2. Verfahren nach Anspruch 1, das auf der Grundlage der Art der ermittelten Verletzung die Bestimmung einer Kontrollbeobachtung zur Überprüfung der Richtigkeit der Art der festgestellten Verletzung umfasst.

3. Verfahren nach Anspruch 2, das die Bestimmung der Kontrollbeobachtung auf der Grundlage mangelnder Klarheit oder einer Widersprüchlichkeit, der beim Durchgang durch den Entscheidungsbaum begegnet wird, umfasst.

4. Verfahren nach einem der vorgenannten Ansprüche, das eine Kennzeichnung beispielsweise durch einen Buchstaben, ein Symbol oder eine Farbkennzeichnung der festgestellten Verbandzusammenstellung umfasst.

5. Gerät zur Bestimmung einer Verbandzusammenstellung für eine bestimmte Verletzung in einer Notsituation, das audiovisuelle Hilfsmittel zum interaktiv sprachgesteuerten Durchlauf durch einen Entscheidungsbaum umfasst zur
○ Bestimmung der Art der Verletzung auf der Grundlage einer visuellen Beobachtung einer verletzten Person (Symptome);
o Bestimmung der zu treffenden Maßnahmen auf der Grundlage der identifizierten Verletzung;
o Bestimmung des erforderlichen Verbandmaterials auf der Grundlage der identifizierten Maßnahme; und
o Bestimmung auf der Grundlage des so identifizierten erforderlichen Verbandmaterials von mindestens einer Verbandzusammenstellung, die zumindest das erforderliche Verbandmaterial umfasst.

6. Verfahren nach Anspruch 5, das umfasst:
- ein Mittel zur audio- und/oder visuellen Darstellung, um dem Anwender eine Reihe von Fragen aus einem Entscheidungsbaum zu stellen; und
- Eingabemöglichkeiten, beispielsweise eine Taste oder ein Mikrofon, um einem Anwender die Möglichkeit zu geben, Fragen zu beantworten, zu welchem Zweck der Entscheidungsbaum geladen worden ist oder in dem Gerät vorhanden ist.

7. Vorrichtung nach Anspruch 5 oder 6, worin das audiovisuelle Mittel ein Computer oder ein Palmtop ist, die eingerichtet sind, um einen Leistungserbringer interaktiv sprachgesteuert durch einen Entscheidungsbaum zu führen zur Bestimmung eines für eine bestimmte Verletzungsart geeigneten Verbandmaterials oder einer Verbandzusammenstellung.

8. Vorrichtung nach Anspruch 7, worin der Entscheidungsbaum in oder auf einem wiederverwendbaren Datenspeicher oder Datenträger wie beispielsweise einer SD-Karte gespeichert wird.

9. Vorrichtung nach einem der Ansprüche 5-8, die zur Durchführung von zumindest einem Trainingsmodus und einem Unternehmermodus eingerichtet ist.

10. Vorrichtung nach einem der Ansprüche 5-9, mit eingebautem Erste-Hilfe-Set und/oder einem Defibrillator.

## Revendications

1. Procédé pour définir quel jeu de bandages est approprié à une blessure donnée dans une situation d'urgence, consistant :
- à fournir une aide audiovisuelle interactive à commande vocale comportant un arbre de décision afin de :
○ définir le type de blessure sur la base de l'examen visuel d'une personne blessée (symptômes) ;
○ définir l'acte à poser sur la base du type de blessure identifié ;
○ définir la matière de pansement requise sur la base de l'acte identifié ; et
○ définir, sur la base de la matière de pansement requise ainsi identifiée, au moins un jeu de bandages qui comprend au moins la matière de pansement requise.

2. Procédé selon la revendication 1, consistant :
- sur la base du type de blessure constaté, à définir un examen de contrôle pour vérifier l'exactitude du type de blessure constaté.

3. Procédé selon la revendication 2, consistant à définir l'examen de contrôle sur la base d'un manque de clarté ou d'une incohérence rencontrée en parcourant l'arbre de décision.

4. Procédé selon l'une quelconque des revendications précédentes, consistant à fournir une indication, par exemple une lettre, une icône ou une couleur, du jeu de bandages défini.

5. Dispositif pour définir quel jeu de bandages est approprié à une blessure donnée dans une situation d'urgence, comprenant des aides audiovisuelles pour parcourir un arbre de décision de façon interactive par commande vocale afin de :
○ définir le type de blessure sur la base de l'examen visuel d'une personne blessée (symptômes) ;
○ définir l'acte à poser sur la base du type de blessure identifié ;
○ définir la matière de pansement requise sur la base de l'acte identifié ; et
o définir, sur la base de la matière de pansement requise ainsi identifiée, au moins un jeu de bandages qui comprend au moins la matière de pansement requise.

6. Dispositif selon la revendication 5, comprenant :
- un moyen de présentation sonore et/ou visuelle pour soumettre à un utilisateur une série de questions provenant d'un arbre de décision, et
- des moyens de saisie, par exemple un bouton ou un microphone, pour permettre à un utilisateur d'entrer des réponses aux questions, but dans lequel l'arbre de décision a été chargé ou est présent dans le dispositif.

7. Dispositif selon la revendication 5 ou 6, dans lequel l'aide audiovisuelle est un ordinateur ou un ordinateur de poche, configuré pour guider un dispensateur de soins de façon interactive par commande vocale dans un arbre de décision afin de définir quelle matière de pansement ou quel jeu de bandages est approprié(e) à un type donné de blessure.

8. Dispositif selon la revendication 7, dans lequel l'arbre de décision est stocké dans une mémoire ou sur un support de stockage réutilisable tel(le) qu'une carte SD.

9. Dispositif selon l'une quelconque des revendications 5 à 8 configuré pour exécuter au moins un mode « formation » et un mode « entreprise ».

10. Dispositif selon l'une quelconque des revendications 5 à 9 intégré à une trousse de premiers secours et/ou à un défibrillateur.
